# EUROPEAN PATENT SPECIFICATION

(11) **EP 1 353 928 B1**
(45) Date of publication and mention of the grant of the patent: **27.12.2006**
(21) Application number: 02710298.7
(22) Date of filing: 21.01.2002
(51) Int. Cl.: C07D 495/04

(54) **PROCESS FOR PREPARING CLOPIDOGREL**
VERFAHREN ZUR HERSTELLUNG VON CLOPIDOGREL
PROCEDE DE PREPARATION DE CLOPIDOGREL

(30) Priority: 24.01.2001 IN MU008401; 22.10.2001 US 54101
(43) Date of publication of application: 22.10.2003
(73) Proprietor: CADILA HEALTHCARE LIMITED, Ahmedebad 380 015, Gujarat (IN)
(72) Inventor: PANDEY, Bipin, Ahemdabad-380 015, Gujarat (IN); LOHRAY, Vidya Bhushan, Ahmedabad 380 015, Gujarat (IN); LOHRAY, Braj Bhushan, Ahmedabad 380 015, Gujarat (IN)
(74) Representative: Spencer, Michael David
(86) International application number: PCT/IN2002/000012
(87) International publication number: WO 2002/059128

(56) References cited:
- US-A- 4 529 596
- ALAIN BURGOS, JOHN M. HERBERT AND IAIN SIMPSON: "ortho-Metalaton/Chlorination of Benzoic Acid Derivatives. Preparation of [benzene-U-13C]-rac-Clopidogrel ([benzene-U-13C]-rac-SR25990C)" J. LABELLED COMPD. RADIOPHARM., vol. 43, no. 9, 2000, pages 891-8, XP008008850
- JERRY MARCH: "Advanced Organic Chemistry" 1992 , WILEY & SONS , NEW YORK XP002215119 198280 The HCN can be generated in situ from acetone cyanohydrin (see 6-49), avoiding the use of poisonous HCN. page 812 The addition of HCN ... This is an equilibrium reaction. page 964 Frequently it is the bisulfite addition product that is treated with CN- page 965 6-51 The addition of HCN to C=N and CN Bonds page 966
- NAJER ET AL.: "Nitriles, amides, thioamides et acides alpha-phenyle alpha-tertioaminoacötiques" BULL. SOC. CHIM. FR., 1958, pages 1189-92, XP002215118
- J. AM. CHEM. SOC., vol. 92, 1972, page 6676,
- "Organic Syntheses (6)" 905

## Description

### FIELD OF THE INVENTION

The present invention relates to a process for the preparation of thieno[3,2-c]pyridine derivatives of general formula (I), in either racemic or optically active (+) or (-) forms and their salts, wherein X, the substituent on benzene ring represents either a hydrogen or halogen atom such as fluorine, chlorine, bromine or iodine.

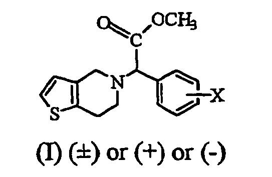

Preferably, X represents 2-chloro.

The present invention also describes a process for preparing the compounds of general formula (II), in either racemic or optically active (+) or (-) forms and their salts, where X, the substituent on benzene ring represents either a hydrogen or halogen atom such as fluorine, chlorine, bromine or iodine.

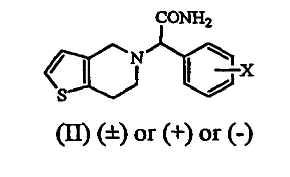

Preferably X represents 2-chloro. These compounds are useful intermediates to prepare compounds of general formula (I).

The compounds represented by formulae (I) and (II) have one asymmetric carbon and hence, to obtain optically active compounds of formula (I) or of formula (II), option available is either to resolve the racemic intermediate/final product or use an optically active intermediate.

### BACKGROUND OF THE INVENTION

Thieno[3,2-c]pyridine derivatives disclosed in FR 2,215,948, FR 2,530,247 and FR 2,612,929, are pharmacologically active and have significant anti-aggregating and antithrombotic properties. One such example is 'Clopidogrel', (*S*)-(+)-(2-chlorophenyl)-(6,7-dihydro-4*H*-thieno[3,2-c]pyridin-5-yl) acetic acid methyl ester and its pharmaceutically acceptable salts. Later, it was found that the biological activity resides only with (*S*)-(+)-stereoisomer (US 4,847,265). As 'Clopidogrel base' is an oily liquid, in order to prepare a convenient formulation, the base is converted into a pharmaceutically acceptable salt. Suitable salts of 'Clopidogrel base' can be formed with taurocholate, hydrobromide and sulfuric acid.

Reported methods for synthesising compounds of general formula (I) (US 4529596, GB 0420706 and GB 0466569), use α-halophenylacetic acid derivatives, which are lacrimatory and irritant in nature. Processes to synthesize such compounds involve multiple steps, and have other drawbacks due to the chemicals/reagents used, which are usually difficult to handle, scale-up and are unfavorable to human health as well as the environment. Moreover, overall yields of these processes range from poor to average. Various other synthetic approaches found in literature, involve expensive or hazardous chemicals, which do not significantly improve the yield of the desired product.

Recently, radiolabelled (bezene-U-¹³C) racemic(±)-Clopidogrel has been prepared as a standard for metabolic studies in an overall yield of 7 % using orthometalation/chlorination of benzoic acid derivative (Burgas et al, J. Labelled Cpd Radiopharm, 43, 591-893 (2000)). This document discusses synthesis of Clopidogrel in the presence of acetone cyanohydrine. Various of the strategies are disclosed in: WO 98/51681, WO 98/51682, WO 98/51689, WO 99/18110, US 4,876,362, US 5,036,156, US 5,132,435, US 5,139,170, US 5,204,469 and US 6,080,875.

Recently, a new polymorph of Clopidogrel bisulfate (named as form II) has been disclosed in patent application (WO 99/65915), which has a melting point of 176 ± 3 °C. It also mentions that the compound disclosed in the earlier US patent (US 4,847,265), had a different melting point of about 184 ± 3 °C (now referred as, form I). It has been shown that both the polymorphs have distinct and characteristic XRD and IR spectrum.

Consequently, the present invention aims to provide an inexpensive and commercially viable process to prepare compounds of formula (I) in good yields.

### SUMMARY OF THE INVENTION

The present invention provides a process for the preparation of thieno[3,2-c]pyridine derivatives, represented by the general formula (I), in either racemic or optically active (+) or (-) forms and their salts, wherein X represents either hydrogen or halogen atom such as fluorine, chlorine, bromine or iodine.

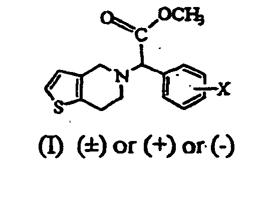

An embodiment of the present invention provides a process to manufacture (*S*)-(+)-(2-chlorophenyl)-(6,7-dihydro-4*H*-thieno[3,2-c]pyridin-5-yl)acetic acid methyl ester as bisulfate salt, i.e. Clopidogrel bisulfate. Preferably, X represents 2-chloro.

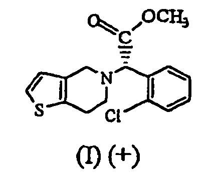

Furthermore, the present invention provides a process for the preparation of compounds of formula II (2-halophenyl)-(6,7-dihydro-4*H*-thieno[3,2-c]pyrid-5-yl)acetamide either in racemic or as optically active (+) or (-) forms and their salts. Preferably, X represents 2-chloro.

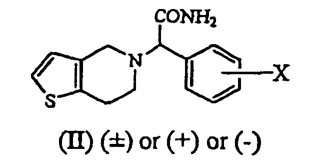

The dextro isomers of compounds of formula (II) with suitable purity or their salts, are useful intermediates for the synthesis of (+)-Clopidogrel bisulfate.

Another embodiment of the present invention provides a process for the preparation of compound of formula (III), (2-halophenyl)-(6,7-dihydro-*4H*-thieno[3,2-c]pyrid-5-yl)acetic acid, in racemic (±) or in either of the optically active (+) or (-) form, and their salts. Preferably, the halogen is chlorine.

A further embodiment of the present invention provides a process for the preparation of a compounds of formula (IV), (±)-(2-halophenyl)-(6,7-dihydro-4*H*-thieno[3,2-c]-pyrid-5-yl)acetonitrile, and their salts. Preferably, the halogen is chlorine.

### DETAILED DESCRIPTION OF THE INVENTION

The present invention provides a process to prepare compounds of formula (I), in either racemic or optically active (+) or (-) forms and their salts, where X represents either hydrogen or a halogen atom such as fluorine, chlorine, bromine or iodine. More particularly, the present invention provides a process to prepare Clopidogrel bisulfate.

The process to prepare compounds of formula (I) or their salts, uses compounds of formula (II) 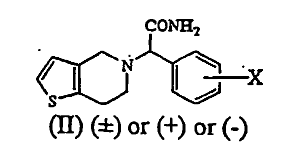 or their salts, in either racemic or optically active (+) or (-) forms, as outlined in Scheme 1.

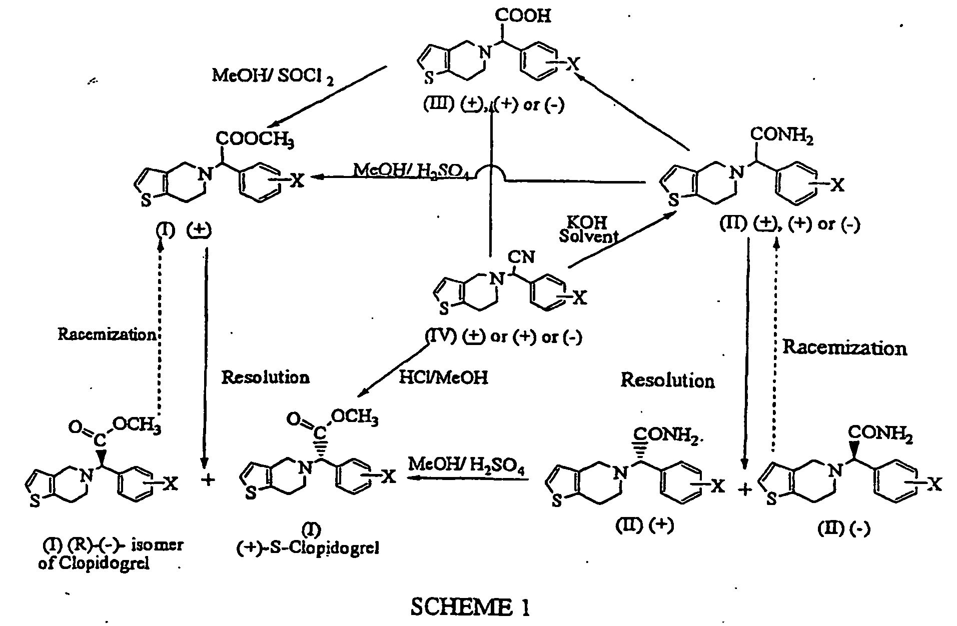

Each intermediate in Scheme 1 has one chiral center. Hence, to prepare an optically active product, such as compound represented by formula (I), particularly Clopidogrel and its salt, it is possible to use an optically active intermediate from the first step onwards.

The present invention provides a process for the preparation of compounds of formula (I) and their salts as shown in scheme 1, which comprises:
1. preparing compound of formula (TV), (±)-(2-halophenyl)-(6,7-dihydro-4*H*-thieno[3,2-c]pyrid -5-yl)acetonitrile as described in Scheme 2, i.e. via Strecker reaction;
2. resolving, if desired, the racemic mixture of compound of formula (IV) into its optically active (+) and (-) stereoisomers; and recycling the unwanted stereoisomer into the process by racemization;
3. transforming the compound of formula (IV) in either racemic or optically active (+) or (-) form or its salt, into the compound of formula (II), (±)-(2-halophenyl)-(6,7-dihydro-4*H-*thieno[3,2-c]pyrid-5-yl)acetamide or optically active corresponding (+) or (-) form, based upon starting material used;
4. resolving, if desired, the racemic compound of formula (II) - into its optically active (+) and (-) stereoisomers; and recycling the unwanted stereoisomer into the process by racemization;
5. transforming the compound of formula (II), either in racemic or optically active (+) and (-) form or its salt, into either optically active or racemic compound of formula (I), (±)-(2-halophenyl)-(6,7-dihydro-4*H*-thieno[3,2-c]pyrid-5-yl)acetate methyl ester, in racemic or optically active (+) and (-) form and its salt, based upon starting material used;
6. further resolving and/or transforming the racemic/optically active compound of formula (I) into their pharmaceutically acceptable salts and/or, liberating the racemic or optically active compound of formula (1) from its salts.

Alternatively, either of the compounds of formulae (IV) or (II), either racemic or optically active (+) or (-) form can be transformed into corresponding compounds of formula (III); which can then be converted into corresponding compound of formula (I).

The compound of formula (IV) in racemic or optically active (+) or (-) forms can be directly converted into corresponding compound of formula (I).

Optionally, suitable acid addition salts of the intermediates of formula II, III and IV may be used in the above mentioned processes. Suitable acids used may be selected from acetic, benzoic, fumaric, maleic, citric, tartaric, gentisic, methanesulfonic, ethanesulfonic, benzenesulfonic, p-toluenesulfonic, camphor sulfonic, hydrochloric, sulfuric, hydrobromic acids and the like.

Another aspect of the present invention is to provide a process for the preparation of a novel intermediate of formula (IV) and its salts.

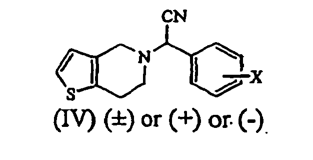

Yet another aspect of the process of invention includes preparation of intermediate described by general formula (IV) and as depicted in the Scheme 2, by Strecker reaction, using a secondary amine (Organic Synthesis Collective Volume III, page no. 275).

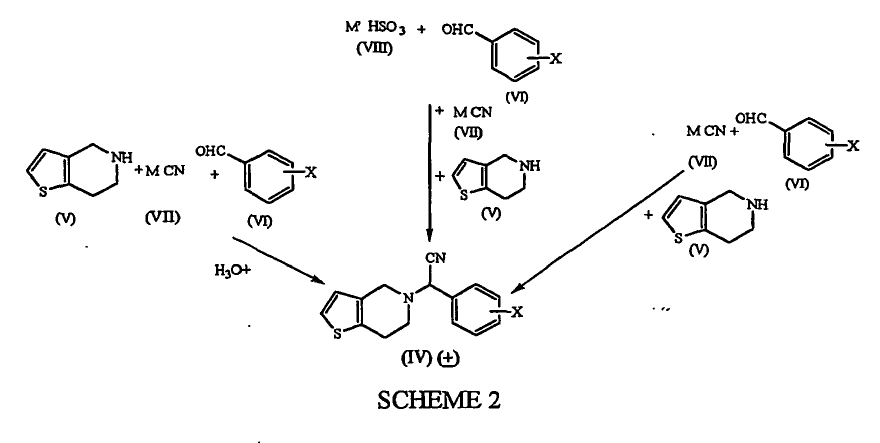

The process to prepare compounds of formula (IV) includes, reacting amine of formula (V) or its salt, with a cyanide derivative of the general formula (VII), wherein M represents either alkali metals such as Na, K, Li, or H, trimethylsilyl (TMS) and the like; with 2-chlorobenzaldehyde of formula (VI). The synthesis of amine or its salt having formula (V) is described in FR 2608607.

The above reaction can be carried out in various ways. A few such methods are outlined in Scheme 2 shown above. Initially, amine of formula (V), or its salt, is reacted with cyanide (VII), wherein M is as defined earlier, followed by addition of 2-chlorobenzaldehyde (VI). Alternatively, 2-chlorobenzaldehyde (VI) is treated with cyanide of formula (VII), wherein M is as defined earlier, and the intermediate cyanohydrin is further reacted with amine of formula (V) or its salt. In an alternative method, 2-chlorobenzaldehyde of formula (VI) is added to hydrogen sulfite derivative of formula (VIII) wherein M' represents Na, K, Li and the like; followed by reaction with cyanide of formula (VII), wherein M is as defined earlier, and finally amine of formula (V) or its salt in an *in situ* reaction. Irrespective of the variations in the reaction methodology, the yield of resultant intermediate (IV) obtained is comparable.

The preferred method involves, addition of 2-chlorobenzaldehyde of formula (VI) to hydrogen sulfite derivative of formula (VIII). The salt formed is treated with cyanide of formula (VII), and finally with an amine of formula (V) or its salt in presence of suitable reagent and solvents.

Suitable reagents includes acid catalysts, such as glacial acetic acid (Synthesis, 1989, 616-618), hydrochloric acid, sulfuric acid, methanesulfonic acid, trifluoroacetic acid, polyphosphoric acid and the like.

Suitable solvents can be hydrophilic solvents, either protic or aprotic, includes water, (C₁-C₄) alcohol, tetrahydrofuran, dimethyl formamide, DMSO, dioxane, 1,2-dimethoxyethane, acetic acid, propionic acid and the like, or a mixture of solvents thereof The preferred solvent is a mixture of solvents and water in varying ratio. The more preferred reaction medium includes a mixture containing water and (C₁-C₄)alcohol in a ratio varying between 1:1 and 1:10.

When the reaction is carried out in aprotic or hydrophobic solvent, a phase transfer catalyst and a biphasic solvent system is necessary. Suitable phase transfer catalyst used in such a case may be tetrabutyl ammonium halide, benzyltrimethylammonium halide, and the like.

During the reaction, certain additives may be added. Such suitable additives may be cyclo[(S)-histidine-(S)-phenyl alanine] and the like.

The reaction temperature may range from -30 °C to reflux temperature of the solvent(s) used. The preferred temperature ranges from 0 °C to 100 °C, and more preferably, from 40 °C to 80 °C. However, when HCN (g) (Scheme 2, Intermediate VII, M = H) is used the required temperature is in the range of about -30 °C to -10 °C.

This reaction may be carried out in the absence or presence of an inert atmosphere such as N₂, He or Ar. The duration of the reaction may vary from 1 hrs to 3 days, more specifically 2 hrs to 2 days.

It is preferable to react a compound of formula (V), hydrogen sulfite derivative (VIII), and cyanide derivative (VII) with respect to 2-chloro benzaldehyde (VI) in the ratio preferably between 1 to 1.2 equivalents. The racemic cyano compound (IV) thus obtained, can be resolved into optically active (+) and (-) forms.

The cyano compound (IV) thus obtained can be converted into corresponding acid of formula (III), amide of formula (II) or acid of formula (I) as shown in Scheme 1 (R. C. Larrock, in "Comprehensive Organic Transformations", John Wiley & Sons, Inc, 1999, 2nd Ed., 815 - 818 and references therein).

Yet, another aspect of the present invention is to convert these intermediates II and III, into compounds of formula I, as shown in scheme 1. Each of (±), (+) or (-) the isomer of intermediate of formulae II and III, can be converted into the corresponding isomer of compounds of formula I.

The preferred route to obtain the compound of formula (I), involves conversion of either (±), (+) or (-) isomer cyano compound (IV) and its salts, into amide compound of formula (II), in the presence of suitable acids/base reagents in suitable solvents. Later resolving the amide into optically active (+) or (-) form or its salt and the optically active amide is being converted into optically active ester of formula (I) in presence of suitable catalyst and reagent.

The reaction to convert cyano compound of formula (TV) into amide compound of formula (II) may be carried out in presence of reagents, which include acid or a base. Suitable acids which may be used are, acetic acid, *p*-toluenesulfonic acid, trifluoroacetic acid, chloroacetic acid and the like or anhydrous alcoholic or aqueous solution of mineral acids such as sulfuric acid, HCl, HBr and the like. A base is preferred whenever the starting material is a racemic mixture. Suitable base which may be used are lithium hydroxide, sodium hydroxide, potassium hydroxide, potassium *tert*-butoxide, or mixtures thereof, preferably alkali metal hydroxides. Along with alkali metal hydroxides, excess of hydrogen peroxides or metal peroxides may also be used in the above reaction. Suitable solvent/s for the above reaction may be aqueous, polar or protic solvents such as water, (C₁-C₄)alcohol, acetone, acetic acid, dimethyl formamide, THF, DMSO, dioxane, DME and the like or mixtures thereof, preferably solvent consists of water, methanol or *tert*-BuOH or mixture of these solvents, in a ratio varying between 1:1 to 1:10.

The temperature ranges from 20 °C to 250 °C, preferably, from 50 °C to 150 °C. The reagents used in the above process can be in the range from 0.01 to 1.2 moles equivalents. The reaction may be carried out in the absence or presence of an inert atmosphere such as N₂, He or Ar. The reactions under the basic conditions are preferably under inert atmosphere. The duration of reaction may range from 1/2 hr to 5 days, preferably from 2 hrs to 2 days.

The amide of formula (II), in either racemic or optically active (+) or (-) form, or their salt, can be converted to corresponding methyl ester of formula (I), in presence of at least one equivalent of methanol and acid, in suitable solvent.

Suitable acids which can be used include acetic acid, polyphosphoric acid, *p-*toluenesulfonic acid, trifluoroacetic acid, chloroacetic acid, or mineral acids, which includes, sulfuric acid, HCl, HBr and the like, which could be in different forms like acid dissolved in alcohol, anhydrous acids dissolved or saturated in alcohol and alcohol used may be methanol. The preferable acid is concentrated sulfuric acid in the 1 to 50 equivalent ratio. Suitable solvents for the above transformation may be polar or protic solvent such as hydrophilic solvents including methanol, acetone, acetic acid, THF, DMSO, dioxane, DME and the like or mixtures thereof The preferable solvent consists of methanol at least in one equivalent and may be in large excess such that it acts as a solvent. Sometimes inert cosolvent, such as toluene, xylene etc. can also be used.

The temperature ranges from 20 °C to 250 °C, preferably from 50 °C to 150 °C. The reaction may be carried out in the absence or presence of an inert atmosphere such as N₂, He or Ar. The duration of reaction may range from 3 hrs to 5 days, preferably from 4 hrs to 2 days.

It is possible to convert compound of formula (IV) in either racemic or optically active (+) or (-) form or its salt is converted into the corresponding acetic acid derivative of formula (III) in presence of suitable solvent and reagent. Suitable solvent/s may be aqueous or alcoholic in nature. Suitable reagents for the above reaction include acids as well as bases.

It is also possible to convert the cyano compound of formula (IV) in either racemic or optically active (+) or (-) form or its salts, directly into methyl ester of formula (I), in presence of at least one equivalent of acid and at least one equivalent of methanol in suitable solvents according to methods known in the literature.

The acid of formula (III) in either racemic or optically active (+) or (-) form or its salts can be converted into corresponding methyl ester of formula (I), in presence of suitable reagent in suitable solvents and at least one equivalent of methanol.

Suitable reagent which can be used include, thionyl chloride, acid chlorides such as pivaloyl chloride, alkylchloroformates like ethyl or methyl chloroformates and other such reagents which activate the COOH group, in a 1:1 equivalent ratio. Suitable solvent for the above transformations may be polar or protic solvent such as, methanol, acetone, dimethylformamide, THF, DMSO, dichloromethane, dichloroethane, dioxane, DME and the like or mixtures thereof. The preferable solvent consists of methanol in at least one equivalent and may be in large excess such that it acts as a solvent. The temperature ranges from 20 °C to 250 °C, preferably from 50 °C to 150 °C.

The reagents used in above process may range from 0.01 moles to equimolar ratios. The reaction may be carried out in the absence or presence of an inert atmosphere such as N₂, He or Ar. The duration of reaction may range from 3 hours to 5 days, preferably from 3 hr to 2 days.

This manufacturing process to prepare the compounds of general formula (I) as shown in scheme 1, has following advantages:
1) It requires less number of steps to prepare the compounds of the formula (I).
2) Simple readily available reagents / chemicals are used.
3) Milder reaction conditions are employed in various steps.
4) It is possible to get chiral/optically active intermediates at every stage (I, II, III, IV, or V)
5) It is possible to racemize the unwanted isomers thereby enhancing efficiency and reducing environmental load.
6) The above factors contribute to improve cost effectiveness of the process described herein.

The compounds of the formulae (I), (II), (III) and (IV) can be resolved by various methods to get optically active compounds of the formulae (I), (II), (III) and (IV), which can give Clopidogrel of desired stereochemistry (R. A. Sheldon, in "Chirotechnology", Marcel Dekker, Inc. NY, Basel, 1993, 173-204 and references therein; A. N. Collins, G. N. Sheldrack and J Crosby, in "Chirality in Industry IT", John Wiley & Sons, Inc, 1997, 81-98 and references therein; E. L. Eliel and S. H. Wilen, in "Stereochemistry of Organic Compound", John Wiley & Sons, Inc, 1999, 297-464 and references therein).

The process of resolution comprises of dissolving the racemic mixture (of formulae I, II, III or IV) in suitable solvent and addition of a suitable chiral reagent. Optionally the medium may contain water about < 5 %. Suitable solvent is selected on the basis whether the diastereomeric salt precipitates out differently. The separation of diastereomeric salt may result either spontaneously or by addition of co-solvent or salting out or evaporation of the solvent or addition of a cosolvent. Alternatively, the separation may result simply by stirring at a suitable temperature in a solvent(s) until one of the salts preferentially precipitate out. Purification of diastereomeric salt is possible by refluxing in a suitable solvent. The free base is liberated from its salt using a suitable base reagent. The diastereomeric salt is dissolved or suspended in a mixture of water and organic solvent and is neutralized with a base under stirring. The free base is obtained after separation of aqueous layer and evaporation of the organic solvent.

The solvents used during the resolution can include solvents or mixtures thereof such as (C₁-C₄) alcohol, (C₁-C₄)ketone, dimethylformamide, ethyl acetate, methyl acetate, methyl ethyl ketone, acetonitrile, propionitrile, THF, dioxane and the like; the solvent used optionally may contain water up to 5 %, but presence of water or its amount is not critical. Suitable temperature range for the resolution includes temperature from 0 °C to reflux temperature of the solvent used, preferably 0 °C to 80 °C. The acid chiral reagents, which can be used to form a diastereomeric salt, include tartaric acid, mandelic acid, lactic acid, camphorsulfonic acid, lactic acid, maleic acid, amino acids and the like.

By repeated crystallization from a suitable solvent, the precipitated salt is enriched in the salt of dextrorotatory isomer of the desired diastereomer to yield a product of constant optical rotation.

Suitable base reagent for the hydrolysis of diastereomeric salt includes sodium carbonate, potassium carbonate, sodium hydrogen carbonate and potassium hydrogen carbonate in aqueous media at temperatures varying between 5 °C to 25 °C.

Finally, the desired salt of compound of formula (II), (III), or (IV); or pharmaceutically acceptable salt of compound of formula (I) can be formed from the corresponding stereoisomer and a suitable acid. The optically pure (*S*)-(+) compound of formula (I), is converted into its bisulfate salt using sulfuric acid 70 % to 98 %, in an appropriate solvent at suitable temperature to afford (+)- Clopidogrel bisufate, polymorph I as desired.

Alternatively, the diastereomers formed may be separated by conventional methods of purification such as fractional crystallization, column chromatography and the like followed by cleavage of salt to give product of desired stereochemistry. It is preferable to use, such a chiral agent, which can selectively form diastereoisomer with either R or S stereoisomer of intermediate I, II, III or IV. The chiral reagent used may be in 0.5 to 1.1 molar ratio.

Determination of the enantiomeric purity of the (+)-dextrorotatory and (-)-laevorotatory enantiomers may be carried through proton NMR spectroscopy with the addition of a chiral rare earth reagents (shift reagents) or by HPLC using a chiral stationary phase as well as through measurement of optical rotation.

The absolute stereochemistry of the diastereomeric salt of II, III or IV compounds may be determined using conventional methods, such as X-ray crystallography. The absolute stereochemistry of chiral compounds can also be determined by comparing it with the reference standards known in literature.

The pharmaceutically acceptable mineral and organic acid salts of optically active enantiomers of Clopidogrel are prepared using various acidic salts, which forms a part of this invention but are not limited to hydrogen sulfates, hydrohalides, taurocholates and the like.

More specifically the present process of invention results in Clopidogrel bisulfate of melting point 184 ±3 °C, which is characteristic of Clopidogrel bisulfate form I. Alternatively, Clopidogrel bisulfate form II can also be prepared by known method (WO 99/65915, FR 98 07464).

The process of this invention also includes the process to recycle the unwanted stereoisomer through racemization. The conditions for racemization of all the intermediates of general formula II, III or IV as well as final product I, involves the similar solvent and catalyst in equimolar quantities. Suitable catalyst is generally a base such as LDA(Lithium diisopropylamide), KOH, NaOH, K⁺-*t*-BuO⁻, NaOMe, NaH, KH and the like. Suitable solvent used during the resolution can include solvents or mixtures thereof such as (C₁-C₄) alcohol, (C₁-C₄)ketone, ethyl acetate, methyl acetate, methyl ethyl ketone, THF, dioxane and the like; the solvent used optionally may contain water up to 5 %. Suitable temperature range for the resolution includes temperature from 0 °C to reflux temperature of the solvent used, preferably 0 ° to 80 °C.

The processes described in the present invention are demonstrated in the examples illustrated below.

### EXAMPLE 1

### (±)-(2-chlorophenyl)-(6,7-dihydro-4H-thieno[3,2-c]pyrid-5-yl)acctonitrile (IV)

To a solution of 8.98 g (86.33 mmole) sodium bisulfite in water (27 mL), o-chlorobenzaldehyde 12.4 g (86.33 mmole) was added resulting in a white precipitate. To the precipitate, 15 g (0.107 moles) of 6,7-dihydro-4*H*-thieno[3,2-c]pyridine was added, followed by addition of 4.4 g (89.7 mmoles) NaCN (dissolved in 15 mL water). The reaction mixture was heated at 40 - 50 °C for 6 hrs and was quenched by pouring in water (50 mL). The mixture was extracted with 2 x 100 mL of ethyl acetate. The organic layer was dried over anhydrous sodium sulfate and the solvent was removed under reduced pressure. The yield of the title product is 24 g (97 %).

The product obtained was characterized using IR spectrum, Mass, ¹³C-NMR and ¹H-NMR, which are as given below :

| | |
|---|---|
| IR spectrum (cm⁻¹) | : 2227 (w, CN group) |
| Mass spectrum (m/z) | : 289.1 (M+H)⁺ |
| ¹³C-NMR (CDCl₃) | : δ 136.46, 132.78, 132.38, 130.69, 130.46, 130.38, 129.90, 126.73, 124.96, 123.01, 115.09, 59.12, 49.30, 47.66, 25.47. |
| ¹H-NMR (CDCl₃) | : δ 7.2-7.7 (4H, m), 7.0 (1H, d), 6.69 (1H, d), 5.32 (1H, s), 3.78 (1H, d), 3.65 (1H, d), 2.8-δ3.0 (4H, m). |

### EXAMPLE-2

### (±)-(2-chlorophenyl)-(6,7-dihydro-4H-thieno[3,2-c]pyrid-5-yl)acetonitrile (IV)

140.5 g (1 mol) ofo-chlorobenzaldehyde and 65 g of (1.01 mol) KCN was added to 3.5 L of methanol. 139.05 g (1 mol) of 6,7-dihydro-4*H*-thieno[3,2-c]pyridine and 190 mL of glacial acetic acid was added to the reaction mixture, which was heated at 60 °C for 20 hrs with stirring. After 8 hrs, precipitate begins to appear and then the reaction mixture was poured in water and extracted with (2 x 25 mL) ethyl acetate. The solvent was removed under reduced pressure and the residue purified as described in Example 1. The yield of product was 187 g (65 %), having melting point = 123 -124 °C.

The product obtained was characterized using IR spectrum, Mass, ¹³C -NMR and ¹H-NMR and was found to be identical to the product obtained in example 1.

### EXAMPLE-3

### (±)-(2-chlorophenyl)-(6,7-dihydro-4H-thieno[3,2-c]pyrid-5-yl)acetonitrile (IV)

27.6 g (266 mmol) of sodium bisulfite was dissolved in 100 mL water, and 38.2 g (271 mmol) o-chlorobenzaldehyde was added after dissolving in 100 mL methanol. A thick white slurry results, which was heated at 60 °C for 1 hour, followed by addition of, 36.97 g (266 mmol) of 6,7-dihydro-4*H*-thieno[3,2-c]pyridine at 40 °C and was stirred for 2 hrs. To this reaction, 17.29 g (266 mmol) of KCN dissolved in 50 mL water was added carefully and heating was continued at 40 °C for 5 - 6 hrs, giving white precipitate. The reaction mixture was worked as described in Example 1 and the yield obtained was 54.6 g (72 %).

The product obtained was characterized using IR spectrum, Mass, ¹³C -NMR and ¹H-NMR and was found out to be identical to the product obtained in example 1.

### EXAMPLE-4

### (±)-(2-chlorophenyl)-(6,7-dihydro-4H-thieno[3,2-c]pyrid-5-yl)acetonitrile (IV)

4.5 g (25.64 mmol) 6,7-dihydro-4*H*-thieno[3,2-c]-5-pyridinium hydrochloride was added (at 10 °C) to solution of potassium cyanide 1.95 g (30 mmol) in 2 mL of ice-cold water, and was followed by dropwise addition of 5 mL concentrated hydrochloric acid at 0 °C. After addition of HCl, 3.3 g (23.47 mmol) o-chlorobenzaldehyde dissolved in 50 mL methanol, was added dropwise. Later the reaction mixture was kept at room temperature for 3 days and afterwards at 50 °C for 3 hrs. The pH was adjusted to 7.5 - 8.0 by dropwise addition of NH₄OH and the product was extracted with ethyl acetate (2 x 50 mL). The solvent was dried over sodium sulfate and evaporated under reduced pressure. The amount of product obtained was 1.67 g (18 %) with melting point = 122 -124 °C.

The product obtained was characterized using IR spectrum, Mass, ¹³C -NMR and ¹H-NMR and was found to be identical to the product obtained in example 1.

### EXAMPLES 5

### (±)-(2-chlorophenyl)-(6,7-dihydro-4H-thieno[3,2-c]pyrid-5-yl)acetonitrile (IV)

6.25 g (44.46 mmol) o-chlorobenzaldehyde was dissolved in 60 mL of toluene. To the same flask, 10 mL glacial acetic acid and 1.24 g (4.343 mmol) cyclo[(S)-histidine-(S)-phenyl alanine] were added, and the temperature was lowered to -25 °C. This was followed by addition of 7 g (50.35 mmol) 6,7-dihydro-4*H*-thieno[3,2-c]pyridine and the reaction flask was purged with HCN gas (at the rate of 30 bubbles/min) at -25 °C for 6 hrs and later was stirred at 31 °C for 2 days. The solvent was removed under vacuum, the residue was purified as mentioned in example 1. The amount of product obtained was 5.5 g (43 %) with melting point = 124 -125 °C.

The product obtained was characterized using IR spectrum, Mass, ¹³C -NMR and ¹H-NMR and was found to be identical to the product obtained in example 1.

### EXAMPLE 6

### (±)-(2-chlorophenyl)-(6,7-dihydro-4H-thieno[3,2-c]pyrid-5-yl)acetonitrile (IV)

As described in Example 5, the adduct was formed between 104.3 g (1 mol) sodium bisulfite and 144.39 g (1.02 mol) o-chlorobenzaldehyde, to which 150 g (1.078 mol) 6,7-dihydro-4H-thieno[3,2-c]pyridine was added at 31 °C to and stirred for 1 hr. 102 g TMS-CN was added dropwise and temperature was maintained at 31 °C for 6 hrs, resulting in a white product, which was isolated and purified according to the procedure in example 1. The yield obtained was 30 g (10 %) having melting point about 123-124 °C.

The product obtained was characterized using IR spectrum, Mass, ¹³C -NMR and ¹H-NMR and was found to be identical to the product obtained in example 1.

### EXAMPLE 7

### (±)-(2-chlorophenyl)-(6,7-dihydro-4H-thieno[3,2-c]pyrid-5-yl)acetonitrile (IV)

To 19.5 g (250 mmol) KCN dissolved in water (20 mL) and 43 g (250 mmol) 6,7-dihydro-4*H*-thieno[3,2-c]pyridine was added, followed by dropwise addition of 50 ml concentrated hydrochloric acid. After the addition was complete, a solution of 33 g (230 mmol) o-chlorobenzaldehyde in 100 mL methanol was added dropwise and stirred for 8 hrs at 31 °C. The pH of the reaction mixture was adjusted to 7.5 - 8.0 using NH₄OH and the product was extracted with ethyl acetate (2 x 500 mL) and washed with water (2 x 500 mL), brine (500 mL) and was dried over sodium sulfate. The solvent was evaporated under reduced pressure to give of 50 g (74 %) the product with melting point of about 123-125 °C.

The product obtained was characterized using IR spectrum, Mass, ¹³C -NMR and ¹H-NMR and was found to be identical to the product obtained in example 1.

### EXAMPLE 8

### (±)-(2-chlorophenyl)-(6,7-dihydro-4H-thieno[3,2-c]pyrid-5-yl)acetonitrile(IV)

To the solution of 35.5 g (342 mmol) sodium bisulfite in 35 mL water, 49.1 g (349 mmol) of o-chloro benzaldehyde was added dropwise, whereupon solid adduct forms instantaneously. To this 50 g (284.9 mmol) 6,7-dihydro-4*H*-thieno[3,2-c]pyridine hydrochloride was added and refluxed for 5 hrs. The usual workup and purification gave 40 g (40 %) of the product.

The product obtained was characterized using IR spectrum, Mass, ¹³C -NMR and ¹H-NMR. The product obtained was found to be identical to the product obtained in example 1.

### EXAMPLE 9

### (±)-(2-chlorophenyl)-(6,7-dihydro-4H-thieno[3,2-c]pyrid-5-yl)acetonitrile (IV)

123.83 g (880 mmol) o-chlorobenzaldehyde and 44 g (897 mmol) sodium cyanide were added to in 100 mL of methanol and water (1:1) mixture. To this 150 g (1070 mmol) 6,7-dihydro-4H-thieno[3,2-c]pyridine was added, followed by the addition of 10 mL concentrated hydrochloric acid and was stirred for 2 days at 31 °C temperature. The pH of reaction mixture was adjusted to 7.5 - 8.0 using NH₄OH. The product was extracted with ethyl acetate (2 x 50 mL) and washed with water (2 x 50 mL), brine (50 mL) and was dried over sodium sulfate, and isolated as given in Example 1. The amount of white solid product obtained was 33 g (13 %), which was characterized as usual.

### EXAMPLE 10

### (±)-(2-chlorophenyl)-(6,7-dihydro-4H-thieno[3,2-c]pyrid-5-yl)acetamide (II)

48 g (0.166 mol) (±)-(2-chlorophenyl)-(6,7-dihydro-4*H*-thieno[3,2-c]pyrid-5-yl)acetonitrile was suspended in 240 ml *t*-BuOH, and add 18.26 g (0.332 mol) of KOH was added in one lot with stirring. The reaction mixture was refluxed at 80 - 82 °C for 3 hr., then cooled to 30°C, 240 mL of water was added and stirred for 20 min. The-lower aqueous layer was separated and fresh 720 mL chilled water (5 - 10 °C) was added slowly in 15 min. The product was extracted with ethyl acetate (2 x 50 mL) and washed with water (2 x 50 mL), brine (50 mL) and it was dried over sodium sulfate, followed by isolation by evaporating the solvent under reduced pressure. On treatment with hexane solid was obtained. The yield of product was 48 g (94 %).

The product obtained was characterized using IR spectrum, Mass, ¹³C -NMR and ¹H-NMR, which are as given below;

| | |
|---|---|
| IR spectrum (cm⁻¹) | : 1656 (s, C=O group), 2333.7 (N-H) |
| Mass spectrum (m/z) : | 307.2 (M+H)⁺ |
| ¹³C-NMR (CDCl₃) | : δ 173.82, 135.32, 133.42, 133, 130.27, 129.99, 129.4, 126.98, 125.18, 122.98, 69.12, 50.77, 49.10, 25.82. |
| ¹H-NMR (CDCl₃) | : δ 7.4 - 7.5 (4H, m), 7.24 (1H, d), 7.0 (1H, s), 6.66 (1H, d), 6.0 (1H, s), 4.88 (1H, d), 3.61 (2H, q), 2.88 (4H, m). |
| Melting point | : 125 - 127 °C |

### EXAMPLE 11

### (±)-(2-chlorophenyl)-(6,7-dihydro-4H-thieno[3,2-c]pyrid-5-yl)acetamide (II)

100 mg (0.3466 mmole) (±)-(2-chlorophenyl)-(6,7-dihydro-4H-tbieno[3,2-c]pyrid-5-yl)acetonitrile of formula (TV) prepared according to Example 1-9, was dissolved in the 5 mL HCl and 1 mL trifluoroacetic acid and 5 mL *t*-butanol was added and refluxed for 4 hrs. After the reaction was complete, the product was isolated as mentioned in example 11. The yield of the product was 40 mg (38 %).

The product obtained was characterized using IR spectrum, Mass, ¹³C -NMR and ¹H-NMR and was found to be identical to the product obtained in example 10.

### EXAMPLE 12

### (±)-(2-chlorophenyl)-(6,7-dihydro-4H-thieno-[3,2-c]pyrid-5-yl)acetamide (II)

In 100 mg (0.346 mmole) (±)-(2-chlorophenyl)-(6,7-dihydro-4H-thieno[3,2-c]pyrid-5-yl) acetonitrile of formula (IV) (prepared according to Example 1 to 9, 5 mL formic acid and 5 mL concentrated hydrochloric acid was added and the reaction mixture was stirred for 48 hrs at 25-30 °C, and later refluxed at approx. 100 °C for 6 hrs and was stirred for 8 days at 25-30 °C. After completion of reaction, the reaction mixture was worked up as mentioned in Example 10. The yield of the product was 50 mg (47 %).

The product obtained was characterized using IR spectrum, Mass, ¹³C -NMR and ¹H-NMR. The product obtained was identical to the product obtained in example 10.

### EXAMPLE 13

### (±)-(2-chlorophenyl)-(6,7-dihydro-4H-thieno[3,2-c]pyrid-5-yl)acetamide (II)

200 mg (0.694 mmole) (±)-(2-chlorophenyl)-(6,7-dihydro-4H-thieno[3,2-c]pyrid-5-yl)acetonitrile of formula (IV) prepared according to Example 1-9, was added to 5 ml HBr and 5 ml H₂O and was stirred for 72 hrs at room temperature. The reaction mixture was then refluxed for 11 hrs at 100 °C and the product was isolated as mentioned in example 10. The yield of the product was 50 mg (47%).

The product was characterized using IR spectrum, Mass, ¹³C -NMR and ¹H-NMR and was found to be identical to the product obtained in example 10.

### EXAMPLE 14

### (±)-(2-chlorophenyl)-(6,7-dihydro-4H-thieno[3,2-c]pyrid-5-yl)acetamide (II)

1 g (3.47 mmole) (±)-(2-chlorophenyl)-(6,7-dihydro-4*H*-thieno[3,2-c]pyrid-5-yl)acetonitrile, was dissolved in 5 mL H₂SO₄(50 %). To the reaction mixture 0.405 g anhydrous NaCl was added and reflux the reaction at for 2-3 hrs. At the end of reaction, the product was isolated as described in Example 10. The yield of the product was 600 mg (57 %).

The product was characterized using IR spectrum, Mass, ¹³C -NMR and ¹H-NMR and was found to be identical to the product obtained in example 10.

### EXAMPLE 15

### (±)-(2-chlorophenyl)-(6,7-dihydro-4H-thieno[3,2-c]pyrid-5-yl)acetamide (II)

1 g (3.47 mmole) (±)-(2-chlorophenyl)-(6,7-dihydro-4*H*-thieno[3,2-c]pyrid-5-yl)acetonitrile was dissolved in the 5 g (5 mmol) HClO₄ and 10 mL H₂O was added to the reaction mixture. The reaction mixture was refluxed at 105 °C for 7 hrs and later stirred at room temperature for 12 hrs. The pH was raised until 10-12, using 10 %w/v NaOH solution and later extracted with 50 mL dichloromethane. The organic layer was isolated, and washed with water and evaporated under reduced pressure. The residue was treated with hexane to give 50 mg (47 % yield) solid, having melting point 125-127 °C.

The product obtained was characterized using IR spectrum, Mass, ¹³C -NMR and ¹H-NMR and was found to be identical to the product obtained in example 10.

### EXAMPLE 16

### (±)-(2-chlorophenyl)-(6,7-dihydro-4H-thieno[3,2-c]pyrid-5-yl)acetamide (II)

1 g (3.47 mmol) of (±)-(2-chlorophenyl)-(6,7-dihydro-4*H*-thieno[3,2-c]pyrid-5-yl)acetonitrile was suspended in 10 mL *t*-BuOH, and 277 mg (6.925 mmol) crushed NaOH was dumped under stirring. The reaction mixture was refluxed at 80 - 82 °C for 4 hrs, followed by cooling to room temperature. The product was isolated by extraction with ethyl acetate. The organic extract was evaporated, and oily material left behind upon hexane treatment yields 400 mg solid (38 %).

The product obtained was characterized using IR spectrum, Mass, ¹³C -NMR and ¹H-NMR and was found to be identical to the product obtained in example 10.

### EXAMPLE 17

### (±)-(2-chlorophenyl)-(6,7-dihydro-4H-thieno[3,2-c]pyrid-5-yl)acetamide (II)

3 g (0.01 mol) (±)-(2-chlorophenyl)-(6,7-dihydro-4*H*-thieno[3,2-c]pyrid-5-yl)acetonitrile was suspended in 50 mL acetone. To this solution 1 g (0.023 mol) NaOH dissolved in 10 mL water was added, followed by 5 mL H₂O₂ (0.05 mol, 30 % w/v) in one lot and was refluxed for 3 hrs. The reaction mixture was cooled to room temperature and 5 mL H₂O₂ (0.05 mol, 30 % w/v) was added again and stirred for 12 hrs. The product was isolated by addition of excess water and extracted with 1 L ethyl acetate. The organic extract was evaporated to give 2.8 g crude oily material, which after treatment with hexane, yields 1 g solid (31 %).

The product obtained was characterized using IR spectrum, Mass, ¹³C -NMR and ¹H-NMR and was found to be identical to the product obtained in example 10.

### EXAMPLE 18

### (±)-(2-chlorophenyl)-(6,7-dihydro-4H-thieno[3,2-c]pyrid-5-yl)acetamide (II)

To 10 g (0.0346 mol) (±)-(2-chlorophenyl)-(6,7-dihydro-4*H*-thieno[3,2-c]pyrid-5-yl)acetonitrile suspended in 30 ml isopropyl alcohol, 3.9 g (0.0589 mol) crushed KOH (85 %) was added slowly and the mixture was warmed, and 120 ml water was added. The pH of aqueous layer was brought to 7 using dilute hydrochloric acid. The white solid precipitate was filtered and washed with 100 mL water. The yield of product was 9 g (85 %) with melting point: 122 °C.

The product obtained was characterized using IR spectrum, Mass, ¹³C -NMR and ¹H-NMR and was found to be identical to the product obtained in example 10.

### EXAMPLE 19

### (±)-(2-chlorophenyl)-(6,7-dihydro-4H-thieno[3,2-c]pyrid-5-yl)acetamide hydrogen sulfate salt (II)

2 g (6.48 mole) of (±)-(2-chlorophenyl)-(6,7-dihydro-4*H*-thieno[3,2-c]pyrid-5-yl)acetamide of formula (IV), was dissolved in 10 mL acetone. To the reaction mixture 1 mL of sulfuric acid was added and was stirred for 0.5 hr. Later 5 mL of diethyl ether was added and stirred overnight at room temperature to obtain a salt. The salt 2 g (76 %) was isolated by filtration and washed with acetone.

| | |
|---|---|
| Melting point : | 199.1 °C |
| IR spectrum : | 1682.8, 3116 (cm ⁻¹). |

### EXAMPLE 20

### (±)-(2-chlorophenyl)-(6,7-dihydro-4H-thieno[3,2-c]pyrid-5-yl)acetic acid (III)

To 100 mg (0.3466 mmole) (±)-(2-chlorophenyl)-(6,7-dihydro-4*H*-thieno[3,2-c]pyrid-5-yl) acetonitrile was dissolved in 2 mL *t*-butanol and add 1.5 mL HCl was added. The reaction mixture was refluxed for 9 hrs at 100 °C. In the end, after the completion of reaction, water was added and pH was brought to 4 with 10 % KOH solution. The product was extracted with 5 mL dichloromethane and proceeded as given in the earlier example. The yield was 40 mg (38 %).

The product obtained was characterized using IR spectrum, Mass, ¹³C -NMR and ¹H-NMR, which are as given below;

| | |
|---|---|
| IR spectrum (cm⁻¹) | : 1637.5 (s, C=O group), 3399.3 (O-H) |
| Mass peaks (m/z) | : 308.1 (M+H)⁺ |
| ¹H-NMR (δ ppm) | : δ 7.22 - 7.89 (4H, m), δ 7.11 - 7.12 (1H, d), δ 6.61 - 6.63 (1H, d), δ 3.57 - |
| | 3.67, (2H, d), δ 4.13 (2H, s), δ 3.32 - 3.42 (2H, s). |

### EXAMPLE 21

### (±)-(2-chlorophenyl)-(6,7-dihydro-4H-thieno[3,2-c]pyrid-5-yl)acetic acid (III)

5 g (17.33 mmole) (±)-(2-chlorophenyl)-(6,7-dihydro-4*H*-thieno[3,2-c]pyrid-5-yl)acetonitrile was added to 100 mL HCl and the mixture was stirred for 2 days and later refluxed for 15 hrs. in the end the reaction mixture was dumped in water, and the pH was raised to 4 using 10 % KOH. The product was extracted with 2 L dichloromethane, washed with water, and the organic layer was evaporated to obtain the residue. Usual purification of the residue gave 2 g of solid (38 %).

The product obtained was characterized using IR spectrum, Mass, ¹³C -NMR and ¹H-NMR. The product obtained was found identical to the product obtained in example 20.

### EXAMPLE 22

### (±)-Methyl (2-chlorophenyl)-(6,7-dihydro-4H-thieno[3,2-c]pyrid-5-yl)acetate (I)

Mix 10 g (32.62 mmole) (±)-(2-chlorophenyl)-(6,7-dihydro-4*H*-thieno[3,2-c]pyrid-5-yl) acetamide (prepared according to example 15), with 19.8 g (161.7 mmole) DMFDMA (Dimethyl formamide dimethylacetal) in 100 mL methanol. The mixture was refluxed at 70 °C for 14 hr. Later the reaction mixture was quenched in water and extracted with ethyl acetate. The organic extract was evaporated under reduced pressure to give 5 g of oily product (48 %). This oil was used without any further treatment to prepare salts of ester (I).

### EXAMPLE 23

### (±)-Methyl (2-chlorophenyl)-(6,7-dihydro-4H-thieno[3,2-c]pyrid-5-yl)acetate (I)

15 g (0.0490 mol) of (±)-(2-chlorophenyl)-(6,7-dihydro-4*H*-thieno[3,2-c]pyrid-5-yl)acetamide, was dissolved in 105 mL methanol, with stirring. To the above solution, 45 mL (0.823 mol) of concentrated sulfuric acid (98 %) was added dropwise at room temperature and over a period of 1.5 hour. Later the reaction mixture was refluxed at 80 °C for 26 hour, followed by distillation of methanol. To the residue left behind, 200 mL ethyl acetate was added at temperature between 0 °C to 5 °C along with stirring. After the addition 99 g (1.764 mol) KOH dissolved in 300 mL water was added to the reaction and was stirred for 0.5 hour. Finally, the reaction mixture was filtered and allowed to stand. The organic layer was isolated and dried over anhy. Na₂SO₄. The solvent was evaporated to obtain oily product. Yield was 10 g (64 %).

### EXAMPLE 24

### (±)-Methyl (2-chlorophenyl)-(6,7-dihydro-4H-thieno[3,2-c]pyrid-5-yl)acetate hydrogen sulfate salt (I)

10 g of (±) Methyl (2-chlorophenyl)-(6,7-dihydro-4*H*-thieno[3,2-c]pyrid-5-yl)acetate prepared according to Example 22, was dissolved in 100 mL of ice-cold acetone and 2 mL concentrated sulfuric acid was added at 0 °C to 5 °C. The crystalline white to off white product formed was isolated by filtration and washed with 20 mL of acetone. The product obtained was dried in vacuum oven at 50 °C. The yield of titled product was 7.2 g (56 %).

### EXAMPLE 25

### (±)-Methyl (2-chlorophenyl)-(6,7-dihydro-4H-thieno[3,2-c]pyrid-5-yl)acetate (I)

2 g (0.00652 mol) (±) (2-chlorophenyl)-(6,7-dihydro-4*H*-thieno[3,2-c]pyrid-5-yl)acetamide and 2 mL (0.0308 mol) methane sulfonic acid and 20 mL methanol were mixed and the solution was refluxed at 85 °C for 12 hrs. The excess of solvent was removed under reduced pressure. The pH was adjusted to about 9 with aq. solution of sodium bicarbonate at 0 °C, and the product was extracted with 70 mL ethyl acetate. The combined organic extracts were dried over anhy. Na₂SO₄ and concentrated. The residue obtained was purified by column chromatography using hexane : ethyl acetate as eluent. The product thus obtained was concentrated, was stored under nitrogen atmosphere, and kept in refrigerator before converting into salt. The yield of titled product was 0.419 g (20 %).

### EXAMPLE 26

### (±)-Methyl (2-chlorophenyl)-(6,7-dihydro-4H-thieno[3,2-c]pyrid-5-yl)acetate (I)

1 g (0.00326 mol) (±) (2-chlorophenyl)-(6,7-dihydro-4*H*-thieno[3,2-c]pyrid-5-yl)acetamide was dissolved in 20 mL methanol and refluxed at 85 °C. During the reflux 10 mL polyphosphoric acid was added dropwise over a period of 1 hr and refluxing was continued for 6 hour. The excess of solvent was removed under reduced pressure. To the residue, 50 mL ethyl acetate was added at 0 °C and the reaction mixture was made basic with aq. NaHCO₃, upto pH 9. Out of the two phases separated, the organic layer was isolated, dried over anhydrous Na₂SO₄ and concentrated. The residue obtained was purified by column chromatography using hexane: ethyl acetate (9:1) as eluent. The product obtained was stored under nitrogen atmosphere and kept in refrigerator before converting into salt. The yield of titled product was 310 mg (30 %).

### EXAMPLE 27

### (±)-Methyl (2-chlorophenyl)-(6,7-dihydro-4H-thieno[3,2-c]pyrid-5-yl)acetate (I)

1 g (0.00326 mol) (±) (2-chlorophenyl)-(6,7-dihydro-4*H*-thieno[3,2-c]pyrid-5-yl)acetamide was added to 2 mL toluene at 0 °C along with stirring, followed by dropwise addition of 1 mL titanium tetrachloride, and the reaction was stirred at 0 °C for 1 hour. Later 18 mL of methanol was added, and then the reaction was stirred for 36 hour at 29 °C and later reflux for 3 hrs. The solvent was distilled under reduced pressure and the residue was added to aq. sodium carbonate at 0 °C. the product was extracted with 20 mL ethyl acetate, and the organic layer was isolated, dried over anhy. Na₂SO₄, concentrated and purified by column chromatography using hexane : ethyl acetate as eluent. The product obtained was stored under nitrogen atmosphere and kept in refrigerator before converting into salt. The yield of titled product was 0.157 g (12-15 %).

### EXAMPLE 28

### (±)-Methyl (2-chlorophenyl)-(6,7-dihydro-4H-thieno[3,2-c]pyrid-5-yl)acetate (I)

To 5 g (16.31 mmol) (±) (2-chlorophenyl)-(6,7-dihydro-4H-thieno[3,2-c]pyrid-5-yl)acetamide, 25 mL POCl₃ was added under stirring. The contents were refluxed until amide was completely consumed (approx. 4 hr). Afterwards 20 mL methanol and 5 mL concentrated H₂SO₄ were added and stirred at room temperature for 1 hour. Later the reaction mixture was refluxed for 1 hour. The reaction mixture was quenched with aq. Na₂CO₃ at 0 °C, and extracted with 200 mL ethyl acetate. The organic layer was isolated, dried over anhy. Na₂SO₄, concentrated and purified by column chromatography using hexane : ethyl acetate as eluent. The product obtained was stored under nitrogen atmosphere and kept in refrigerator before converting into salt. The yield of titled product was 0.943 g (18%).

### EXAMPLE 29

### (±)-Methyl (2-chlorophenyl)-(6,7-dihydro-4H-thieno[3,2-c]pyrid-5-yl)acetate (I)

1 g (0.00326 mol) (±) (2-chlorophenyl)-(6,7-dihydro-4H-thieno[3,2-c]pyrid-5-yl)acetamide was dissolved in 10 mL methanol. The reaction mixture was saturated with HCl (g) at 0 °C, was stirred at room temperature for 4 hrs, and later was refluxed for 6 hrs. The solvent was removed under reduced pressure. To the residue, 10 mL of ethyl acetate and aqueous NaHCO₃ until pH was 9 (at 0 °C) were added. The organic layer was isolated, dried over anhy. Na₂SO₄, evaporated under reduced pressure. The residue was further purified by column chromatography using hexane : ethyl acetate as eluent. The product obtained was stored under nitrogen atmosphere and kept in refrigerator before converting into salt. The yield of titled product was 0.188 g (18 %).

### EXAMPLE 30

### (±)-Methyl (2-chlorophenyl)-(6,7-dihydro-4H-thieno[3,2-c]pyrid-5-yl)acetate (I)

To 1 g (0.00326 mol) (±) (2-chlorophenyl)-(6,7-dihydro-4*H*-thieno[3,2-c]pyrid-5-yl)acetamide, 3 g (24.2 mmole) DMFDMA (dimethyl formamide dimethyl acetal) and 10 mL methanol were added. The reaction mixture was refluxed at 70 °C for 14 hr, and then poured in water and extracted with ethyl acetate. The combined organic layers are dried over anhy. Na₂SO₄, evaporated under reduced pressure. The residue was further purified by column chromatography using hexane : ethyl acetate (9:1) as eluent. The yield of titled product was 500 mg (48 %).

### EXAMPLE 31

### (S)-(+)-(2-chlorophenyl)-(6,7-dihydro-4H-thieno[3,2-c]pyrid-5-yl)acetamide (II)

a) A solution of 5 g (16.31 mmol) of (±)-(2-chloro-phenyl)-(6,7-dihydro-4*H*-thieno[3,2-c]pyrid-5-yl)acetamide (prepared as given in Examples 10-15) and 4.15 g (16.2 mmol) (1*S*) (+)-camphor-10-sulphonic acid monohydrate in 100 mL acetone was stirred at room temperature for 20 h. Subsequently it was kept for 1 week in a freezer. Few crystals appeared concentration of volume by evaporating solvent under reduced pressure and repeated recrystallization in freezer for few days gave 3.3 g (75 % yield) of (*S*)-2-(2-chloro-phenyl)-(4, 5,6,7-tetrahydrothieno[3,2-c]pyrid-5-yl)acetamide (+)-camphor sulfonic acid salt. The salt was further purified by recrystallization in acetone, until constant specific optical rotation was obtained.
The product obtained was dried suitably. The typical physicochemical characteristics of the product obtained are as follows,

| | |
|---|---|
| Melting range : | 223-225 °C (dec.) |
| SOR | : + 51° (C=1, MeOH) |

b) 3.3 g (*S*)-(+)-(2-chlorophenyl)-(6,7-dihydro-4*H*-thieno[3,2-c]pyrid-5-yl)acetamide 10-D-camphor sulfonic acid salt was added to 20 mL saturated aqueous Na₂CO₃ solution and the product was extracted with 20 mL ethyl acetate. The organic layer was isolated, dried over Na₂SO₄ and concentrated under reduced pressure. The product was obtained crude oil, which upon purification gives, 1.6 g of white crystals (64 %).

The product obtained was characterized by different physico-chemical characteristics including IR spectrum, Mass, ¹³C -NMR and ¹H-NMR, which are as given below;

| | |
|---|---|
| Melting point | : 153 - 154°C |
| SOR | : +41°(C=1,MeOH) |
| Optical purity | : 99.5 % by chiral HPLC column. |
| IR spectrum (cm⁻¹) | : 1656 (s, C=O group), 2333.7 (m, C-N)⁻, 3357.9 (s, N-H str). |
| Mass peaks (m/z) | : 307.2 (M+H)⁺ |
| ¹³C-NMR(CDCl₃) | :δ 173.25, 134.84, 132.87, 132.65, 129.98, 129.56, 129.0, 126.72, 124.87, 122.64, 68.65, 50.43, 48.73, 25.28. |
| ¹H-NMR (CDCl₃) : | δ 7.4 - 7.5 (4H, m), 7.07 (1H, d), 7.06 (1H, d), 6.66 (1H, d), 6.5 (1H, s), 4.9 (1H, s), 3.6 (2H, q), 2.88 (4H, m). |
| HPLC purity | : 99.85 % |

### EXAMPLE 32

### (S)-(+)-(2-chlorophenyl)-(6,7-dihydro-4H-thieno[3,2-c]pyrid-5-yl)acetamide (II)

a) 2 g (6.5 mmol) of (±)-(2-chloro-phenyl)-(6,7-dihydro-4H-thieno[3,2-c]pyrid-5-yl)acetamide was added to 30 mL acetone. 0.82 g (3.28 mmol) (1S)-(+)-camphor-10- sulfonic acid monohydrate in 10 mL acetone was added to above solution at 15 - 20 °C in 4 hrs. The reaction mixture was stirred for another 5 min, where a few crystals were formed. The solvent was distilled under reduced pressure and then the reaction mixture was kept in cold condition at temperature below 8 °C. The precipitate formed was filtered and washed with solvent. The yield of titled product was 1.2 g (60 %) with melting range = 223-225 °C and [α]_{D} = + 51° (C=1, CH₃OH).
b) To the suspension of 1.1 g of (+)-(S)-(2-chlorophenyl)-(6,7-dihydro-4H-thieno[3,2-c]pyrid-5-yl)acetamide (1*S*)-(+)-camphor-10-sulfonic acid salt in 50 mL water, 50 mL of saturated Na₂CO₃ solution in water was added. The reaction mixture was stirred for some time, and was followed by addition of 100 mL ethyl acetate. The organic layer was isolated, and distilled to get the title product. The amount of product obtained was 600 mg (60 %, based upon the dextroisomer present in the racemic mixture).

The product obtained was characterized by different physico-chemical characteristics, which are as given below;

| | |
|---|---|
| Melting range : | 149 -153 °C |
| [α]_{D} : | + 38° (C=1, CH₃OH) with % ee = 97.24 %. |

### EXAMPLE 33

### (S)-(+)-(2-chloro-phenyl)-(6,7-dihydro-4H-thieno[3,2-c]pyrid-5-yl)acetamide (II)

a) 5 g (16.3 mmol) (±)-(2-chlorophenyl)-(6,7-dihydro-4*H*-thieno[3,2-c]pyrid-5-yl)acetamide was added to 60 mL ethyl acetate. 2 g (8.6 mmol) of (1S)-(+)-camphor-10- sulfonic acid monohydrate was dissolved in minimum amount of water and added to the above solution in one lot, and stirred at 35 - 40 °C for 1 hr. In short time salt forms and was isolated, washed with 50 mL acetone and dried. The amount of product obtained was 1.81 g (36 %).
The product obtained was characterized by different physico-chemical characteristics, which are as given below;

| | |
|---|---|
| Melting range : | 223 - 225 °C |
| [α]_{D} : | + 52.12° (C = 1, CH₃OH). |

b) To the suspension of 1.8 g of (*S*)-(+)-(2-chloro-phenyl)-(6,7-dihydro-4*H*-thieno[3,2-c]pyrid-5-yl) acetamide (1*S*)-(+)-camphor-10-sulfonic acid salt in 100 mL water, 50 mL of sodium bicarbonate solution in water was added. After stirring the mixture, 150 mL ethyl acetate was added. The combined organic layers are distilled off to get title product. The amount of product obtained was 1 g (56 %).

The product obtained was characterized by different physico-chemical characteristics, which are as given below, similar to example 31;

| | |
|---|---|
| Melting range : | 153-154 °C |
| [α]_{D} : | + 42° (C=1, CH₃OH) and with % ee = 100 % (by chiral HPLC column chromatography). |

### EXAMPLE 34

### (R)-(-)-(2-chloro-phenyl)-(6,7-dihydro-4H-thieno[3,2-c]pyrid-5-yl)acetamide (II)

a) 5 g (±)-(2-chlorophenyl)-(6,7-dihydro-4*H*-thieno[3,2-c]pyrid-5-yl)acetamide (I) (0.016 mol) was added to 60 mL ethyl acetate and to the solution 2 g (0.0086 mol) (*1R*)-(-)-camphor-10-sulfonic acid monohydrate in 5 mL water was added to it in one lot. Reaction mixture was stirred at ambient temperature for 1 hr. Later the reaction mixture was stored in freezer for 1 week, a few crystals were seen. After, evaporation of solvent at reduced pressure and storing in cold condition the salt precipitated out, which was then filtered and washed with 50 mL acetone. The amount of product obtained was 1.7 g (39 %).
The product obtained was characterized by different physico-chemical characteristics of (*R*)-(-)-(2-chloro-phenyl)-(6,7-dihydro-*4H*-thieno[3,2-c]pyrid-5-yl)acetamide and of its (-)-CSA salt, both which are as given below;

| | |
|---|---|
| Melting range (-)-CSA-(-)acetamide: | 219 - 220 °C |
| [α]_{D} (-)-CSA-(-)acetamide : | - 52.12° (C=1, CH₃OH) |

b) To the suspension of 1.6 g (*R*)-(-)-(2-chloro-phenyl)-(6,7-dihydro-4*H*-thieno[3,2-c]pyrid-5-yl)acetamide (*1R*)-(-)-camphor-10-sulfonic acid salt in 100 mL water, 50 mL of sodium bicarbonate solution in water was added. After stirring the mixture, 150 mL ethyl acetate was added. The organic layer was extracted and combined organic layer was distilled off to get title product. The amount of product obtained was 900 mg (36 %).

The product obtained was characterized by different physico-chemical characteristics, which are as given below;

| | |
|---|---|
| Melting range : | 145-149 °C. |
| [α]_{D} acetamide: | -36.49° (C=1, CH₃OH) |

### EXAMPLE 35

### (S)-(+)-Methyl (2-chlorophenyl)-(6,7-dihydro-4H-thieno[3,2-c]pyrid-5-yl)acetate bisulfate (I)

15 g of (+) (*S*)-(2-chloro-phenyl)-(6,7-dihydro-4*H*-thieno[3,2-c]pyrid-5-yl)acetamide (0.0489 mol), was converted into corresponding ester as given in example 23. The ester can be converted into its hydrogen sulfate salt, according to the procedure given in example 24. The amount of product obtained was 7 g (44.5 %), with melting point = 184 - 185 °C.

The melting point, IR spectrum and XRD of the product obtained resembles that of product obtained in EP 281459 and US 4847265 i.e. now referred as form I polymorph of Clopidogrel bisulfate (WO 99/65915).

The product obtained was characterized by different physico-chemical characteristics, as given below;

| | |
|---|---|
| [α]_{D} : | + 55°(C = 1, CH₃OH) |
| Melting point : | 185°C±2°C |
| IR spectrum : | 2980, 1755, 1224, 1175 and 840 respectively with the respective % of percentage ransmittance of approximately: 45; 16; 19; 15; 45. |

XRD was found matching with the form I, XRD reported in WO 99/65915.

### EXAMPLE 36

### (R)-(-)-Methyl (2-chlorophenyl)-(6,7-dihydro-4H-thieno[3,2-c]pyrid-5-yl)acetate bisulfate (I)

Using 5 g (*R*)-(-)-(2-chloro-phenyl)-(6,7-dihydro-*4H*-thieno[3,2-c]pyrid-5-yl)acetamide obtained in the above example 34, can be converted into ester as given in Example 23. Later the ester can be converted into hydrogen sulfate salt as given in Example 24. The amount of product obtained was 3.01 g (44 %).

The product obtained was characterized by different physico-chemical/purity characteristics, as given below;

| | |
|---|---|
| [α]_{D} : | - 52 ° (C = 1, CH₃OH) |

### (S)-(+)-Methyl (2-chlorophenyl)-(6,7-dihydro-4H-thieno[3,2-c]pyrid-5-yl) acetate bisulfate (I)

a) 10 g (0.0185 mol) (*1S*)-(+)-camphor-10-sulfonic acid salt of (+)-(2-chlorophenyl)-(6,7-dihydro-4*H*-thieno[3,2-c]pyrid-5-yl)acetamide was dissolved in 40 ml methanol at 0 °C. To the solution 15 mL (0.28 mol) of conc. H₂SO₄ was added dropwise within 1.5 hr. Gradually the reaction temperature was increased, and it was refluxed for 26 hrs. In the end, the solvent was distilled off under reduced pressure. The residue left behind was mixed with 120 mL of ethyl acetate and the pH was adjusted between 9 to 10 using aq. sodium carbonate for extraction. The organic layer was isolated, dried over anhydrous Na₂SO₄, and evaporated under vacuum. The product obtained was purified by column chromatography using hexane : ethyl acetate (9: 1) as eluent. The combined eluent was evaporated at reduced pressure to yield 5.76 g (97 %) of product.
b) 2 g (*S*)-(+)-Methyl (2-chlorophenyl)-(6,7-dihydro-4H-thieno[3,2-c]pyrid-5-yl)acetate was converted into bisulfate salt according to the procedure given in Example 24. The product obtained was 2.2 g (84 %) of product and found identical to that obtained in example 35.

### EXAMPLE 38

### Polymorph I of (+)-Methyl (2-chlorophenyl)-(6,7-dihydro-4H-thieno[3,2-c]pyrid-5-yl) acetate bisulfate salt (I)

52.5 mL of ice-cold acetone was added to 17.5 g (+)-Methyl (2-chlorophenyl)-(6,7-dihydro-4*H*-thieno[3,2-c]pyrid-5-yl)acetate while stirring at 0 °C. 3.6 mL of concentrated H₂SO₄ was added dropwise and temperature was kept below 5 °C. 20 mL of acetone was further added and reaction mixture was further stirred for 4 hrs at room temperature. Precipitate was isolated (17 g, 74 %), dried under vacuum at temperature not exceeding 50 °C.

The product obtained was characterized by different physico-chemical characteristics and was found identical to that obtained in example 35, as given below;

| | |
|---|---|
| Specific optical rotation : | + 54° (C = 1, CH₃OH) |
| Melting point : | 185 °C ± 2°C |

IR and XRD were found matching to that reported in the literature.

### EXAMPLE 39

### Polymorph I of (+)-Methyl (2-chlorophenyl)-(6,7-dihydro-4H-thieno[3,2-c]pyrid-5-yl)acetate bisulfate salt (I)

2.1 g (+)-Methyl (2-chlorophenyl)-(6,7-dihydro-4H-thieno[3,2-c]pyrid-5-yl)acetate was added 7.6 mL of acetone to obtain a clear solution. To this solution, 0.887 g of H₂SO₄ (80 %) was added slowly and temperature was maintained around 20 °C under nitrogen atmosphere. Later the reaction mixture was cooled upto -20 °C for 2 hrs and then the temperature was brought to room temperature (20 °C). The reaction mixture was stirred at 20 - 25 °C. Precipitate was isolated (600 mg), dried under vacuum at temperature not exceeding 50 °C.

The product obtained was characterized by different physico-chemical characteristics and was found identical to that obtained in example 35, as given below;

| | |
|---|---|
| SOR (α^{D}) : | + 54.03° (C = 1.89, MeOH) |
| Melting point : | 185 °C ± 1 °C |
| Chiral Purity : | 99.63 % (ee) |

IR and XRD were found matching to that reported in the literature.

### EXAMPLE 40

### Polymorph I of (+)-Methyl (2-chlorophenyl)-(6,7-dihydro-4H-thieno[3,2-c]pyrid-5-yl)acetate bisulfate salt (I)

To 2 g (+)-Methyl (2-chlorophenyl)-(6,7-dihydro-4H-thieno[3,2-c]pyrid-5-yl)acetate was added 5 mL of acetone and stirred at 25-30 °C. The temperature of the reaction mixture was raised from 25 to 65 °C and then kept at 65 °C for 5 min. At temperature 50-52 °C 0.676 g of concentrated H₂SO₄ was added. The reaction mixture was cooled from 52 °C to 5 °C, and additional acetone was added and stirred for 5 min. Later the reaction mixture was stirred at 25 - 30 °C for 12 hrs, the thick precipitate obtained was filtered, washed with 5 mL of acetone and the residue was dried in a vacuum oven. The yield of titled product obtained was 1.27 g (47 %).

The product obtained was characterized by different physico-chemical characteristics and was found identical to that obtained in example 35, as given below;

| | |
|---|---|
| SOR (α^{D}) : | + 54.03° (C = 1.89, MeOH) |
| Melting point : | 185 °C ± 1 °C |
| Chiral Purity : | 99.80 % (ee) |

IR and XRD were found matching to that reported in the literature.

### EXAMPLE 41

### Polymorph I of (+)-Methyl (2-chlorophenyl)-(6,7-dihydro-4H-thieno[3,2-c]pyrid-5-yl)acetate bisulfate salt (I)

To 1.98 g (+)-Methyl (2-chlorophenyl)-(6,7-dihydro-4*H*-thieno[3,2-c]pyrid-5-yl)acetate was added 5 mL of acetone and stirred at 25-30 °C. The temperature of the reaction mixture was raised from 25 to 50 -52 °C and within one lot 0.7 g of concentrated H₂SO₄ (95 %) was added with constant stirring and the reaction mixture was suddenly cooled to 0 °C to -5 °C for 10 min. Later the reaction mixture was stirred at 25 - 30 °C for 12 hrs, the thick precipitate obtained was filtered, washed with 5mL of acetone and the residue (1.6 g, 62 %) was dried in a vacuum oven.

The product obtained was characterized by different physico-chemical characteristics and was found identical to that obtained in example 35, as given below;

| | |
|---|---|
| SOR (α^{D}) : | + 55.96° (C = 1.89, MeOH) |
| Melting point : | 185 °C ± 1 °C |
| Chiral Purity : | 99.85 % (ee) |

IR and XRD were found matching to that reported in the literature.

### EXAMPLE 42

### Racemization of (R)-(-)-(2-chlorophenyl)-(6,7-dihydro-4H-thieno[3,2-c]pyrid-5-yl)acetamide (II)

20 g mixture of laevorotatory (*R*)-(-)-(2-chlorophenyl)-(6,7-dihydro-4*H*-thieno[3,2-c]pyrid-5-yl)acetamide of formula (II) and 11 g of potassium *t*-butoxide in 100 mL of *t-*butanol was stirred at 25 °C to 30 °C for 30 min. After completion of reaction, the reaction mixture was poured in 750 mL cold water to obtain a yellow precipitate. This solid obtained was filtered and dissolved in methylene dichloride. The organic layer was washed with 2 x 100 mL of DM water (2 x 100 mL) and concentrated to give 18 g of the corresponding racemic amide i.e. (±)-2-(2-chlorophenyl)-(6,7-dihydro-4H-thieno[3,2-c]pyrid-5-yl)acetamide (II).

The specific optical rotation of racemic amide (II) was ± 1° (C = 1, CH₃OH).

### EXAMPLE 43

### Racemization of (R)-(-)-(2-chlorophenyl)-(6,7-dihydro-4H-thieno[3,2-c]pyrid-5-yl)acetamide (II)

1 g mixture of laevorotatory (*R*)-(-)-(2-chlorophenyl)-(6,7-dihydro-4*H*-thieno[3,2-c]pyrid-5-yl)acetamide of formula (II) and 1 g of potassium *t*-butoxide in 20 mL of DMSO was stirred at 50 °C to 60 °C for 3 hours. After completion of reaction, the reaction mixture was poured in 150 mL cold water to obtain a yellow precipitate. This solid obtained was filtered and dissolved in methylene dichloride. The organic layer was washed with 2 x 25 mL of DM water (2 x 25 mL) and concentrated to give 0.8 g of the corresponding racemic amide i.e. (±)-2-(2-chlorophenyl)-(6,7-dihydro-4H-thieno[3,2-c]pyrid-5-yl)acetamide.

The specific optical rotation of racemic amide (II) was ± 0.5° (C = 1, CH₃OH).

### EXAMPLE 44

### Racemization of (R)-(-)-(2-chlorophenyl)-(6,7-dihydro-4H-thieno[3,2-c]pyrid-5-yl)acetamide (II)

1 g mixture of laevorotatory (*R*)-(-)-(2-chlorophenyl)-(6,7-dihydro-*4H*-thieno[3,2-c]pyrid-5-yl)acetamide of formula (II) and 250 mg of sodium hydride in dry tetrahydrofuran was stirred at 25 °C to 30 °C for 3 hours. After completion of reaction, the reaction mixture was poured in 150 mL cold water slowly to obtain a yellow precipitate. This solid obtained was filtered and dissolved in methylene dichloride. The organic layer was washed with 2 x 25 mL of DM water (2 x 25 mL) and concentrated to give 0.95 g of the corresponding racemic amide i.e. (±)-(2-chlorophenyl)-(6,7-dihydro-4H-thieno[3,2-c]pyrid-5-yl)acetamide (II).

### EXAMPLE 45

### Racemization of (R)-(-)-(2-chlorophenyl)-(6,7-dihydro-4H-thieno[3,2-c]pyrid-5-yl)acetamide (II)

1 g mixture of laevorotatory (*R*)-(-)-(2-chlorophenyl)-(6,7-dihydro-4*H*-thieno[3,2-c]pyrid-5-yl)acetamide of formula (II) and 21 g of potassium *t*-butoxide in 100 mL of DMSO was stirred at 50 °C to 60 °C for 3 hours. After completion of reaction, the reaction mixture was poured in 750 mL cold water to obtain a yellow precipitate. This solid obtained was filtered and dissolved in methylene dichloride. The organic layer was washed with 2 x 100 mL of DM water (2 x 100 mL) and concentrated to give 0.8 g of the corresponding racemic amide i.e. (±)-(2-chlorophenyl)-(6,7-dihydro-4H-thieno[3,2-c]pyrid-5-yl)acetamide.

### EXAMPLE 46

### (S)-(+)-Methyl (2-chlorophenyl)-(6,7-dihydro-4H-thieno[3,2-c]pyrid-5-yl)acetate (I)

5 g of (+)-(2-chlorophenyl)-(6,7-dihydro-4H-thieno[3,2-c]pyrid-5-yl)acetamide was dissolved in 35 ml methanol and the solution was chilled to 0 to -5 °C. 15 mL (0.28 mole) of conc. H₂SO₄ (98 %) was added slowly in 1 hr maintaining the temperature till -5 °C. After the completion of addition the reaction mixture was stirred at room temperature for 30 min. The solution was refluxed at 60 °C for 36 hrs. The reaction mixture was cooled to room temperature and the solvent was distilled off under reduced pressure. The residue was added to 200 mL chilled water was added and cooled to -5 °C. The pH of reaction mixture was adjusted between 9 to 10 using aq. sodium carbonate. The residue was extracted with 100 mL ethyl acetate. The organic layer was dried over anhydrous Na₂SO₄, and evaporated under vacuum. The combined eluent was evaporated at reduced pressure to yield 3.2 g of purified product purified by column chromatography, using hexane : ethyl acetate (9:1) as eluent.

### EXAMPLE 47

### (S)-(+)- Methyl (2-chlorophenyl)-(6,7-dihydro-4H-thieno[3,2-c]pyrid-5-yl)acetate (I)

5 g of (+)-(2-chlorophenyl)-(6,7-dihydro-4H-thieno[3,2-c]pyrid-5-yl)acetamide was dissolved in 45 ml methanol, followed by 15 mL of toluene. 5 mL (0.28 mol) of conc. H₂SO₄ was instantly added and another 10 mL of conc. H₂SO₄ was slowly added later, while maintaining the temperature at 90 °C. After that, the reaction mixture was refluxed at 90 °C for 13 hrs. The solvent was distilled off under reduced pressure. To the residue, 50 mL of water was added and the pH of reaction mixture was adjusted between 9 to 10 using aq. sodium carbonate. The residue was extracted with 100 mL ethyl acetate. The organic layer was dried over anhydrous Na₂SO₄, and evaporated under vacuum. The combined eluent was evaporated at reduced pressure to yield 2.8 g of crude product purified by column chromatography, using hexane : ethyl acetate (9:1) as eluent.

### EXAMPLE 48

### (S)-(+)-Methyl (2-chlorophenyl)-(6,7-dihydro-4H-thieno[3,2-c]pyrid-5-yl)acetate (I)

5 g of (*S*)-(+)-(2-chlorophenyl)-(6,7-dihydro-4H-thieno[3,2-c]pyrid-5-yl)acetamide was dissolved in 50 ml methanol and the solution was cooled till -15 °C. 15 mL (0.28 mol) of conc. H₂SO₄ (98 %) was added dropwise in 1 hr maintaining the temperature till -15 °C. After the completion of addition the reaction mixture was gradually increased, and stirred at 31 °C for 30 min, and then refluxed at 70 °C for 21 hrs. The solvent was distilled off under reduced pressure and to the residue 50 mL of water was added and stirred for 30 min followed by cooling to -5 °C. The pH of reaction mixture was adjusted between 9 to 10 using aq. sodium carbonate. The residue was extracted with 100 mL ethyl acetate. The organic layer was dried over anhydrous Na₂SO₄, and evaporated under vacuum. The yield was 3.8 g of crude product purified by column, using hexane : ethyl acetate (9:1) as eluent.

### EXAMPLE 49

### (S)-(+)-Methyl (2-chlorophenyl)-(6,7-dihydro-4H-thieno[3,2-c]pyrid-5-yl)acetate (I)

5 g of (+)-(2-chlorophenyl)-(6,7-dihydro-4*H*-thieno[3,2-c]pyrid-5-yl)acetamide was dissolved in 20 ml methanol and the solution warmed to 65 to 70 °C. 15 mL (0.28 mol) of chilled conc. H₂SO₄ (98 %, -15 °C) was added slowly in 1 hr maintaining the temperature till -15 °C. After the completion of addition the reaction mixture was heated at 70 °C for 16 hrs. The solvent was distilled off under reduced pressure and to the residue 50 mL of water was added and stirred for 30 min followed by cooling to -5 °C. The pH of reaction mixture was adjusted between 9 to 10 using aq. sodium carbonate. The residue was extracted with 100 mL ethyl acetate. The organic layer was dried over anhydrous Na₂SO₄, and evaporated under vacuum. The combined eluent was evaporated at reduced pressure to yield 2.8 g of crude product purified by column chromatography, using hexane : ethyl acetate (9:1) as eluent to give 2 g of the titled compound (I) and 1 g of the starting material (S)-(+)- (II).

### EXAMPLE 50

### (S)-(+)-(2-chlorophenyl)-(6,7-dihydro-4H-thieno[3,2-c]pyrid-5-yl)acetamide (II)

a) A solution of 5 g (16.31 mmol) of (±)-(2-chloro-phenyl)-(6,7-dihydro-4*H*-thieno[3,2-c]pyrid-5-yl) acetamide (prepared as given in Examples 10-15) and 4.15 g (16.2 mmol) (1*S*) (+)-camphor-10-sulphonic acid monohydrate in 100 mL acetone was kept warm for 20 hrs followed by storing it in temperature below 10 °C. A few crystals appeared, the mother liquor was concentrated further and repeated recrystallization, followed by storage in cold condition for few days gave 3.1 g (70 % yield) of (*S*)-2-(2-chloro-phenyl)-(4,5,6,7-tetrahydrothieno[3,2-c]pyrid-5-yl)acetamide (+)-camphor sulfonic acid salt. The salt was further purified by recrystallization in acetone, until constant specific optical rotation was obtained.
The product obtained was dried suitably. The typical physicochemical characteristics of the product obtained are as follows:

| | |
|---|---|
| Melting range : | 223-225 ° C (dec.) |
| SOR : | + 52° (C = 1, MeOH) |

b) 3.1 g (*S*)-(+)-(2-chlorophenyl)-(6,7-dihydro-4*H*-thieno[3,2-c]pyrid-5-yl)acetamide 10-D-camphor sulfonic acid salt was added to 20 mL saturated Na₂CO₃ solution and the product was extracted with 20 mL ethyl acetate. The organic layer was isolated, dried over Na₂SO₄ and concentrated under reduced pressure. The product was obtained crude oil, which upon purification gives, 1.5 g of white crystals (60 %).

### EXAMPLE 51

### Chiral removal of (-)-stereoisomer from a mixture containing excess of (+)-Methyl (2-chlorophenyl)-(6,7-dihydro-4H-thieno[3,2-c]pyrid-5-yl)acetate (I)

2 g (0.0173 mole) Methyl (2-chlorophenyl)-(6,7-dihydro-4H-thieno[3,2-c]pyrid-5-yl)acetate (where ee was 80 %) was dissolved in 10 ml acetone and the reaction mixture was stirred for 10 min, followed by reflux. To the reaction mixture, 1.49 g (1S)-(+)-camphor-10-sulfonic acid hydrate in 0.8 mL water was added followed by 1 mL acetone. Then whole reaction mixture was refluxed for 1 hrs and cooled gradually. This was later stirred overnight at room temperature. The clear solution was cooled further at 0 to -5 °C, wherein precipitate was obtained. The salt formed was added to ethyl acetate and water, which was later basified with NaHCO₃, the organic layer was washed with water, concentrated under reduced pressure, to give free base 0.386 g with chiral purity = 99.85 % (+)-isomer (ee = 99.7 %).

### EXAMPLE 52

### Chiral removal of (-)-stereoisomer from a mixture containing excess of (+)-Methyl (2-chlorophenyl)-(6,7-dihydro-4H-thieno[3,2-c]pyrid-5-yl)acetate (I)

2 g (0.0173 mole) Methyl (2-chlorophenyl)-(6,7-dihydro-4H-thieno[3,2-c]pyrid-5-yl)acetate (where ee was 90 %) was dissolved in 10 ml acetone and the reaction mixture was stirred for 10 min, followed by reflux. To the reaction mixture, 1.49 g (1S)-(+)-camphor-10-sulfonic acid hydrate in 0.8 mL water was added followed by 1 mL acetone. Then whole reaction mixture was refluxed for 1 hrs and cooled gradually. This was later stirred overnight at room temperature. The clear solution was cooled further at 0 to -5 °C, wherein precipitate was obtained. The salt formed was added to ethyl acetate and water, which was later basified with NaHCO₃, the organic layer was washed with water, concentrated under reduced pressure, to give free base 0.386 g with chiral purity = 99.85 % (+)-isomer (ee = 99.7 %).

### EXAMPLE 53

### Chiral removal of (-)-stereoisomer from a mixture containing excess of (+)-Methyl (2-chlorophenyl)-(6,7-dihydro-4H-thieno[3,2-c]pyrid-5-yl)acetate (I)

2 g (0.0173 mole) Methyl (2-chlorophenyl)-(6,7-dihydro-4H-thieno[3,2-c]pyrid-5-yl)acetate (where ee was 95 %) was dissolved in 10 ml acetone and the reaction mixture was stirred for 10 min, followed by reflux. To the reaction mixture, 1.49 g (1S)-(+)-camphor-10-sulfonic acid hydrate in 0.8 mL water was added followed by 1 mL acetone. Then whole reaction mixture was refluxed for 1 hrs and cooled gradually. This was later stirred overnight at room temperature. The clear solution was cooled further at 0 to -5 °C, wherein precipitate was obtained. The salt formed was added to ethyl acetate and water, which was later basified with NaHCO₃, the organic layer was washed with water, concentrated under reduced pressure, to give free base 0.386 g with chiral purity = 99.85 % (+)-isomer (ee = 99.7 %).

### EXAMPLE 54

### Resolution of (±)-Methyl (2-chlorophenyl)-(6,7-dihydro-4H-thieno[3,2-c]pyrid-5-yl)acetate (I)

33 g (0.1 mol) (±)-Methyl (2-chlorophenyl)-(6,7-dihydro-4H-thieno[3,2-c]pyrid-5-yl)acetate was dissolved in 200 ml acetone and the reaction mixture was heated to 60 to 70 °C, reflux for 15 min. To the reaction mixture, 25.6 g (1*S*)-(+)-camphor-10-sulfonic acid hydrate dissolved in water was added, wherein formation of salt starts at 60 to 70 °C. After the formation of salt was complete, the reaction mixture was cooled gradually to room temperature and then to 0 °C to 5 °C. The isolation of diastereomeric salt by filtration and washed using acetone, and dried. The yield of the diastereomeric product was 20.5 g (70 %).

The generation of (-) isomer of title compound was carried out using dilute sodium bicarbonate solution and extraction in ethyl acetate, removal of solvent and giving us the title compound 10.9 g (66 %).

### EXAMPLE 55

### Resolution of (±)-(2-chlorophenyl)-(6,7-dihydro-4H-thieno[3,2-c]pyrid-5-yl)acetonitrile (IV)

a) To 5 g (0.0173 mol) (±)-(2-chlorophenyl)-(6,7-dihydro-4*H*-thieno[3,2-c]pyrid-5-yl)acetonitrile dissolved in 100 ml acetone, 4.35 g (0.0174 mole) (1*S*)-(+)-camphor-10-sulfonic acid hydrate in 5 mL water was added in one lot at 60 to 62 °C during addition. The mixture is stirred at 60 °C for 60 hr. the stirring was then continued overnight and later kept in refrigerator for 1 day. The diastereomeric salt precipitated was then filtered to yield 730 mg of product.
b) To 730 mg (±)-(2-chlorophenyl)-(6,7-dihydro-4*H*-thieno[3,2-c]pyrid-5-yl)acetonitrile (1*S*)-(+)-camphor-10-sulfonic acid salt in 20 mL water, 10 mL (5 %) Na₂CO₃ solution added (pH = 10). After stirring the mixture, 100 mL ethyl acetate was added to it organic layer was then added to it. Organic layer was then separated and distilled off to get product. The yield of product was 400 mg (16 %).

| | |
|---|---|
| Specific optical rotation(α^{D}): | + 7.5787 ° (C = 1, DMF). |

## Claims

1. A process for the preparation of compound of formula (I), 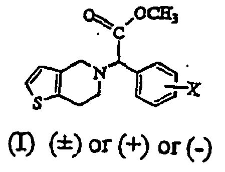 where X represents hydrogen, fluoro, chloro, bromo or iodo, preferably 2-chloro, which comprises:
i) reacting a compound of formula (V) or its salt, 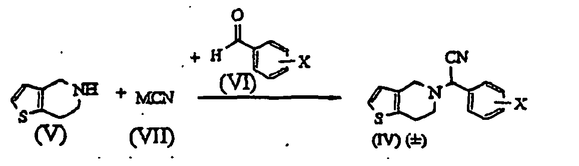 with a cyanide of general formula (VII) where M represents alkali metal, trimethylsilyl, Cu, or hydrogen, with addition of compound of general formula (VI), where X is as defined earlier, to obtain a racemic (±) compound of general formula (IV), where X is as defined earlier,
(ii) reacting a compound of general formula (IV), in (±) form or any of its optically active (+) or (-) forms, 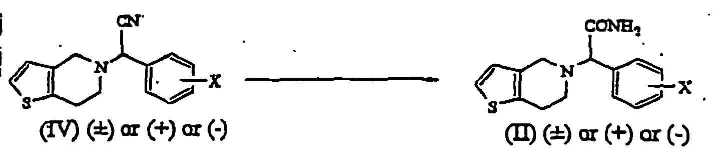 with an acidic or basic reagent to obtain a compound of formula (II) or its salt with retention of configuration,
(iii) reacting a compound of general formula (II), in (±) form or its optically active (+) or (-) forms, 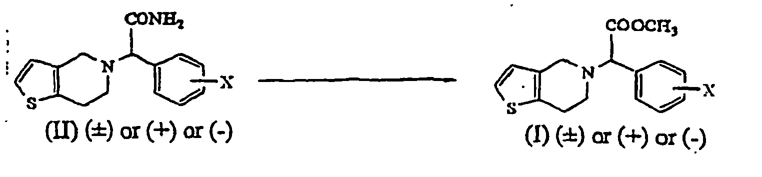 with an acidic reagent in presence of methanol to obtain a compound of formula (I) or its salt, with retention of configuration,
(iv) finally resolving the compound of formula (I) or its salt, if in (±) form, into its optical isomers.

2. A process according to claim 1, also including the steps of resolving a racemic (±) compound of formula (IV) into its optical isomers and/or transforming the racemic compound or its optical isomer into its salt, optionally followed by liberation of the salt from the compound.

3. A process according to claim 1, also including the steps of resolving a racemic (±) compound of formula (II) into its optical isomers and/or transforming the racemic compound or its optical isomer into its salt, optionally followed by liberation of the salt from the compound.

4. A process for the preparation of compound of formula (III) or its salt, where X represents hydrogen, chloro, bromo or iodo, preferably 2-chloro, which comprises, 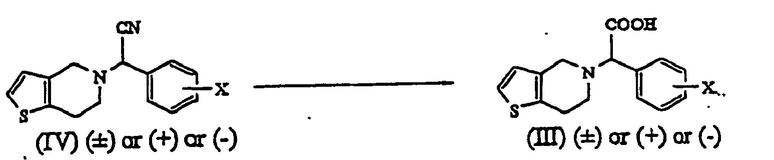
(i) preparing compound of general formula (IV) or its salt, in any of its optical forms by a process according to step i) of claim 1 or 2, in the presence of an acidic/basic catalyst in a suitable solvent, giving the corresponding acid of formula (III) or its salt, with a configuration the same as that of the starting material, and
(ii) if desired, finally the racemic compound of formula (III) or its salt, if in racemic (±) form, are resolved into its corresponding optically pure (+) and (-) forms.

5. A process according to claim 1 or 2, wherein a compound of formula (II) (2-halophenyl)-(6,7-dihydro-4*H*-thieno[3,2-c]pyrid-5-yl)acetamide or its optical active isomers or its salts, where X represents a halogen atom such as chloro, bromo, iodo substituent, more preferably 2-chloro, is prepared by a process comprising, 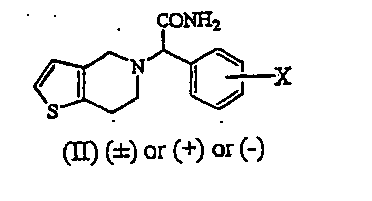
(i) treating the compound of formula (IV) or its salt or in any of its optical forms, with a basic reagent when the starting material is in racemic form or else with an acidic catalyst when the starting material is in optically active form in presence of suitable solvent; and
(ii) finally forming a salt of the racemic compound of formula (II), which is resolved into its corresponding optically pure (+) or (-) forms.

6. A process according to claim 5, which comprises preparation of compound of formula (II) **characterised in that** the basic reagent used is lithium hydroxide, potassium hydroxide, sodium hydroxide and potassium *t*-butoxide.

7. A process according to claim 5, which comprises preparation of compound of formula (II), wherein said acidic catalyst used is selected from the group consisting of acetic acid, *p*-toluenesulfonic acid, trifluoroacetic acid, chloroacetic acid, perchloric acid, formic acid or mineral acids such as anhydrous alcoholic hydrogen halides, or aqueous hydrochloric acid, sulfuric acid, HBr or a mixture thereof.

8. A process according to claim 5, 6 or 7, which comprises preparation of compound of formula (II) wherein the solvent used is water, a (C₁-C₄) alcohol, acetone, acetic acid, dimethylformamide, THF, DMSO, dioxane, DME or mixtures thereof, preferably a mixture of water, methanol or *tert*-BuOH.

9. A process according to any of claims 5 to 8, wherein a compound of formula (II), (±)-(2-chlorophenyl)-(6,7-dihydro-4*H*-thieno[3,2-c]pyrid-5-yl)acetanude, is resolved into its (-) and (+) forms using 1-(*R*) or 1-(*S*)-camphorsulfonic-10-acid or tartaric acid, more particularly 1-(*R*) or 1-(*S*)-camphorsulfonic-10-acid, in the presence of a solvent such as water, acetone, ethyl acetate or a mixture thereof, the salt used optionally being in a molar ratio of 0.5 to 1.1 equivalents.

10. A process according to any of claims 5 to 8, wherein the compound of formula (II), (±)-(2-chlorophenyl)-(6,7-dihydro-4*H*-thieno[3,2-c]pyrid-5-yl)acetarnide, is converted into a corresponding salt selected from D-camphorsulfonate, L-camphor sulfonate, D-tartrate, L-tartrate or hydrogen sulfate.

11. A process according to any of claims 1 to 3 or 5 to 10, wherein said compound of formula (I) is methyl (2-halophenyl)-(6,7-dihydro-4*H*-thieno[3,2-c]pyrid-5-yl)acetate, or its optical active isomers or its salts, where X represents a halogen atom such as chloro, bromo, iodo substituent, more preferably 2-chloro, said process comprising, 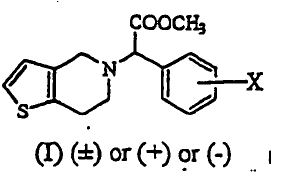
(i) treating the compound of formula (II) or its salt in racemic form or in any of its optical forms, with an acidic reagent in presence of a suitable solvent; and
(ii) forming a salt of the racemic compound of formula (I), which is resolved into its corresponding optically pure (+) or (-) forms.

12. A process according to claim 11, wherein said acidic reagent used is selected from concentrated sulfuric acid in the range from 1 equivalent to 50 equivalents, preferably at 0 °C to 5 °C or at reflux temperature of the solvent used.

13. A process according to claim 11, wherein, the solvent used is methanol in the range of 3 to 30 volumes, along with a cosolvent such as toluene, DMSO or xylene or mixtures thereof.

14. A process according to claim 11, wherein the duration of the treatment with an acidic reagent ranges from 4 hours to 4 days.

15. A process according to claim 11, wherein the reaction is carried out at a temperature in the range from 40 °C to 140 °C.

16. A process according to claim 11, wherein a racemic compound of formula (I), is resolved using 1-(*R*) or 1-(*S*)-camphorsulfonic-10-acid, (*R*) or (*S*)-tartaric acid, more particularly 1-(R) or 1-(*S*)-camphorsulfonic-10-acid, in the presence of a solvent such as water, acetone, ethyl acetate or a mixture thereof.

17. A process according to claim 16, wherein a mixture containing varying ratios of the two enantiomers, (-)-I and (+)-I is chirally enriched to (+)-(I)-stereoisomer, or (-)-I enantiomer is chirally removed from a variable mixture of(-)-I and (+)-I stereoisomers.

18. A process for the preparation of a compound of formula (IV) or its salt, 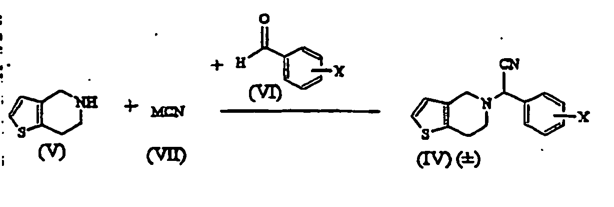 where X represents a halogen atom such as chloro, bromo or iodo, more particularly 2-chloro, which comprises,
i) reacting a compound of formula (V) or its acid addition salt with a cyanide of general formula (VII) wherein the meaning of M is alkali metal, TMS, Cu, and hydrogen, followed by reaction with a halogenobenzaldehyde of the formula (VI), wherein X is a halogen atom, and resolving the product into its optical isomers and, if desired, liberating it from its salt or transforming it into its salt; or
ii) reacting a halogenobenzaldehyde of general formula (VI) wherein the meaning of X is halogen atom with a cyanide of general formula (VII) wherein M is as defined earlier, followed by in situ addition of the compound of formula (V) or its acid addition salt; or
iii) reacting a halogenobenzaldehyde of general formula (VI) wherein the meaning of X is halogen atom, with a hydrogen sulfite of general formula M'HSO₃ (VIII) where M' is sodium, potassium or lithium and finally with a cyanide of general formula (VII), wherein M is as defined earlier, and subsequently reacting *in situ* with the compound of general formula (V) or its acid addition salt; and
iv) finally resolving the resulting compound of formula (IV) or its salt to its optically pure (+) and (-) form of compound of formula (IV) or its salt.

19. A process according to claim 18, wherein the reaction is carried out at temperature between 0 °C and 100 °C, except that when MCN is HCN gas, the reaction temperature can go further down up to -30 °C.

20. A process according to claim 18 or 19, wherein the reaction is carried in an aqueous or a non-aqueous medium containing an acid such as acetic acid or propionic acid, methanol and HCl/MeOH.

21. A process according to any of claims 18 to 20, wherein a catalyst acid such as dry hydrochloric acid, sulfuric acid, *p*-toluenesulfonic acid or acetic acid is used.

22. A process according to any of claims 18 to 21, wherein said compound of formula (IV) (±)-(2-chlorophenyl)-(6,7-dihydro-4*H*-thieno[3,2-c]pyrid-5-yl)acetonitrile, is resolved into (-) and (+) forms using 1(R) or 1(S)-camphorsulfonic-10-acid or tartaric acid, more particularly 1(R) or 1(S)-camphorsulfonic-10-acid, in the presence of acetone, ethyl acetate or a mixture thereof and water.

23. A process according to any of claims 18 to 21, wherein a compound of formula (IV) (±)-(2-chlorophenyl)-(6,7-dihydro-4*H*-thieno[3,2-c]pyrid-5-yl)acetonitrile, in (±) or (-) or (+) forms is converted into its salt, wherein the salt claimed is D-camphorsulfonate, L-camphor sulfonate, D-tarnate, L-tartrate or hydrogen sulfate.

24. A process according to any of claims 18 to 23, wherein racemisation of the (-) isomer of the compound of formula (II) is effected in a mixture which may contain (+) stereoisomer to get (±) compound using a base such as LDA, KOH, NaOH, K-t-BuOH, NaOMe, NaH or KH in a suitable solvent.

25. A process according to claim 1, wherein racemisation if the (-) isomer of compound of formula (I) is effected in a mixture which may contain (+) stereoisomer to get the (±) compound using a base such as LDA, NaOMe, NaH, KH in a suitable solvent.

26. A process according to claim 17, the resolution is carried out in the presence of acetone as a solvent in the range of 5 to 10 volumes, which may contain water up to 5 %.

27. A process according to claim 17, wherein said suitable chiral agent used is in 1:1 molar ratio.

28. A process according to claim 27, wherein the temperature is from 0 °C to the reflux temperature of the solvent.

## Patentansprüche

1. Verfahren für die Herstellung einer Verbindung der Formel (I), 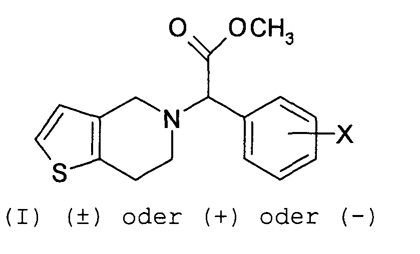 wobei X Wasserstoff, Fluoro, Chloro, Bromo oder Iodo, vorzugsweise 2-Chloro, darstellt, welches umfasst:
i) Umsetzen einer Verbindung von Formel (V) oder ihres Salzes 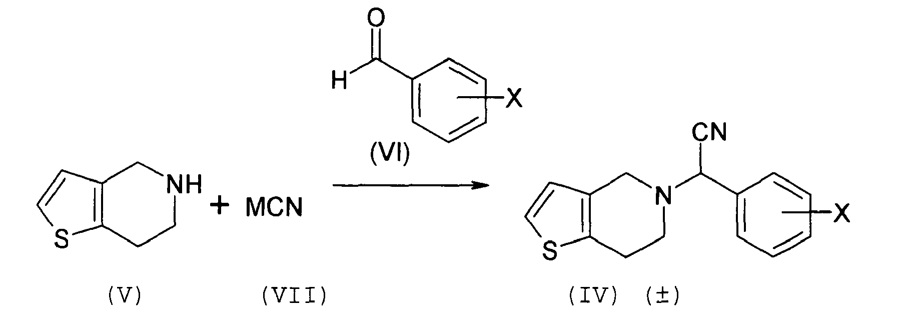 mit einem Cyanid der allgemeinen Formel (VII), wobei M Alkalimetall, Trimethylsilyl, Cu oder Wasserstoff darstellt, unter Addition einer Verbindung der allgemeinen Formel (VI), wobei X wie oben definiert ist, um eine razemische (±)-Verbindung der allgemeinen Formel (IV) zu erhalten, wobei X wie oben definiert ist,
ii) Umsetzen einer Verbindung der allgemeinen Formel (IV) in (±)-Form oder einer ihrer optisch aktiven (+)- oder (-)-Formen, 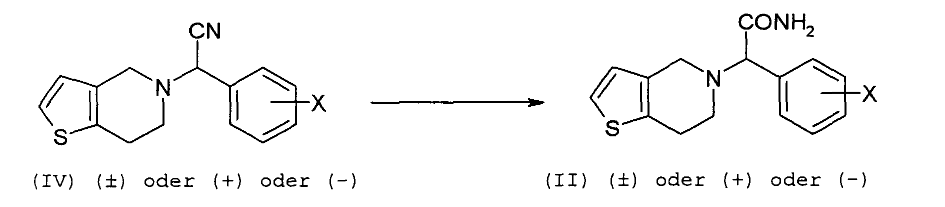 mit einem sauren oder basischen Reagens, um eine Verbindung der Formel (II) oder deren Salz unter Beibehaltung der Konfiguration zu erhalten,
iii) Umsetzen einer Verbindung der allgemeinen Formel (II) in (±)-Form oder einer ihrer optisch aktiven (+)- oder (-) -Formen, 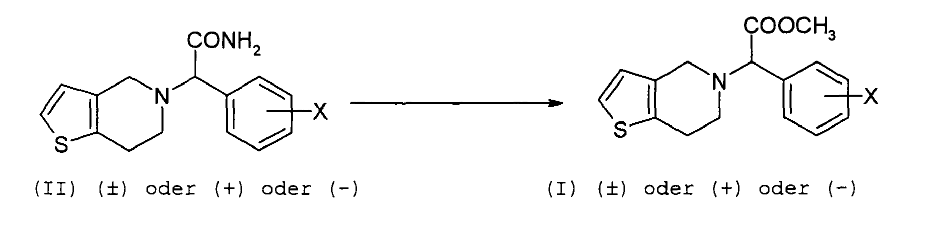 mit einem sauren Reagens in Gegenwart von Methanol, um eine Verbindung der Formel (I) oder deren Salz zu erhalten, unter Beibehaltung der Konfiguration,
(iv) schließlich Auflösen der Verbindung von Formel (I) oder deren Salz, falls in (±) -Form, zu ihren optischen Isomeren.

2. Verfahren nach Anspruch 1, das des Weiteren die Schritte des Auflösens einer razemischen (±)-Verbindung von Formel (IV) in ihre optischen Isomere und/oder das Transformieren der razemischen Verbindung oder ihres optischen Isomers zu ihrem Salz umfasst, wahlweise gefolgt von der Freisetzung des Salzes aus der Verbindung.

3. Verfahren nach Anspruch 1, das des Weiteren die Schritte des Auflösens einer razemischen (±)-Verbindung von Formel (II) in ihre optischen Isomere und/oder das Transformieren der razemischen Verbindung oder ihres optischen Isomers zu ihrem Salz umfasst, wahlweise gefolgt von der Freisetzung des Salzes aus der Verbindung.

4. Verfahren zum Herstellen einer Verbindung nach Formel (III) oder ihres Salzes, wobei X Wasserstoff, Chloro, Bromo oder Iodo, vorzugsweise 2-Chloro, darstellt, welches umfasst, 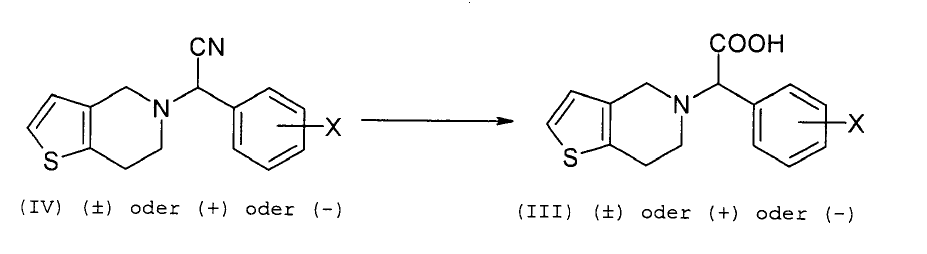
(i) Herstellen einer Verbindung der allgemeinen Formel (IV) oder ihres Salzes in einer ihrer optischen Formen nach einem Verfahren nach Schritt (i) von Anspruch 1 oder 2 in Gegenwart eines sauren/basischen Katalysators in einem geeigneten Lösungsmittel, unter Erhalt der entsprechenden Säure von Formel (III) oder ihres Salzes mit einer Konfiguration, die der des Ausgangsmaterials entspricht, und,
(ii) falls gewünscht, schließlich Auflösen der razemischen Verbindung von Formel (III) oder ihres Salzes, falls in razemischer (±)-Form, zu ihren entsprechenden optisch reinen (+)- und (-)-Formen.

5. Verfahren nach Anspruch 1 oder 2, wobei eine Verbindung von Formel (II) (2-Halophenyl)-(6,7-dihydro-4H-thieno[3,2-c]pyrid-5-yl)acetamid oder deren optisch aktive Isomere oder deren Salze, wobei X ein Halogenatom wie einen Chlor-, Brom-, Iodsubstituenten, mehr bevorzugt 2-Chloro, darstellt, nach einem Verfahren hergestellt wird, welches umfasst, 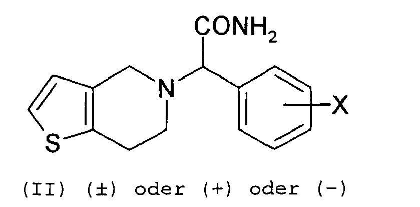
(i) Behandeln der Verbindung von Formel (IV) oder ihres Salzes oder in einer ihrer optischen Formen mit einem basischen Reagens, wenn das Ausgangsmaterial in razemischer Form vorliegt, oder ansonsten mit einem sauren Katalysator, wenn das Ausgangsmaterial in optisch aktiver Form vorliegt, in Gegenwart eines geeigneten Lösungsmittels und
(ii) schließlich Ausbilden eines Salzes der razemischen Verbindung von Formel (II), welche in ihre entsprechenden optisch reinen (+)- oder (-)-Formen aufgelöst wird.

6. Verfahren nach Anspruch 5, das die Herstellung einer Verbindung von Formel (II) umfasst, **dadurch gekennzeichnet, dass** es sich bei dem verwendeten basischen Reagens um Lithiumhydroxid, Kaliumhydroxid, Natriumhydroxid und Kalium-*t*-butoxid handelt.

7. Verfahren nach Anspruch 5, das die Herstellung einer Verbindung von Formel (II) umfasst, wobei der verwendete saure Katalysator aus der Gruppe ausgewählt ist, die aus Essigsäure, p-Toluolsulfonsäure, Trifluoressigsäure, Chloressigsäure, Perchlorsäure, Ameisensäure oder Mineralsäuren wie beispielsweise wasserfreien alkoholischen Wasserstoffhalogeniden oder wässriger Salzsäure, Schwefelsäure, HBr oder einer Mischung davon besteht.

8. Verfahren nach Anspruch 5, 6 oder 7, das die Herstellung einer Verbindung von Formel (II) umfasst, wobei es sich bei dem verwendeten Lösungsmittel um Wasser, einen (C₁-C₄)-Alkohol, Aceton, Essigsäure, Dimethylformamid, THF, DMSO, Dioxan, DME oder Mischungen davon handelt, vorzugsweise eine Mischung aus Wasser, Methanol oder *tert*-BuOH.

9. Verfahren nach einem der Ansprüche 5 bis 8, wobei eine Verbindung von Formel (II), (±)-(2-Chlorphenyl)-(6,7-dihydro-4H-thieno[3,2-c]pyrid-5-yl)acetamid, in Gegenwart eines Lösungsmittel wie Wasser, Aceton, Ethylacetat oder einer Mischung davon in ihre (-)- und (+)-Formen aufgelöst wird, wobei 1-(R)- oder 1-(*S*)-Camphersulfon-10-säure oder Weinsäure, insbesondere 1-(*R*)- oder 1-(*S*)-Camphersulfon-10-säure, verwendet wird, wobei das verwendete Salz wahlweise in einem Molverhältnis von 0,5 bis 1,1 Äquivalenten vorhanden ist.

10. Verfahren nach einem der Ansprüche 5 bis 8, wobei eine Verbindung von Formel (II), (±)-(2-Chlorphenyl)-(6,7-dihydro-4*H*-thieno[3,2-c]pyrid-5-yl)acetamid, in ein entsprechendes Salz umgewandelt wird, ausgewählt aus D-Camphersulfonat, L-Camphersulfonat, D-Tartrat, L-Tartrat oder Hydrogensulfat.

11. Verfahren nach einem der Ansprüche 1 bis 3 oder 5 bis 10, wobei die Verbindung von Formel (I) Methyl-(2-halogenphenyl)-(6,7-dihydro-4*H*-thieno[3,2-c]pyrid-5-yl)acetat oder dessen optisch aktive Isomere oder dessen Salze ist, wobei X ein Halogenatom wie einen Chlor-, Brom-, Iodsubstituenten, mehr bevorzugt 2-Chloro, darstellt, wobei das Verfahren umfasst, 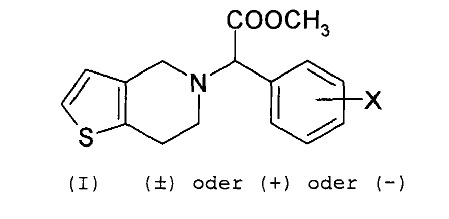
(i) Behandeln der Verbindung von Formel (II) oder ihres Salzes in razemischer Form oder in einer ihrer optischen Formen mit einem sauren Reagens in Gegenwart eines geeigneten Lösungsmittels und
(ii) Ausbilden eines Salzes der razemischen Verbindung von Formel (I), welche in ihre entsprechenden optisch reinen (+)- oder (-)-Formen aufgelöst wird.

12. Verfahren nach Anspruch 11, wobei das verwendete saure Reagens aus konzentrierter Schwefelsäure im Bereich von 1 Äquivalent bis 50 Äquivalenten, vorzugsweise bei 0°C bis 5°C oder bei einer Rückflusstemperatur des verwendeten Lösungsmittels ausgewählt ist.

13. Verfahren nach Anspruch 11, wobei es sich bei dem verwendeten Lösungsmittel um Methanol im Bereich von 3 bis 30 Volumen handelt, zusammen mit einem Begleitlösungsmittel wie Toluol, DMSO oder Xylol oder Mischungen davon.

14. Verfahren nach Anspruch 11, wobei die Dauer der Behandlung mit einem sauren Reagens von 4 Stunden bis 4 Tage reicht.

15. Verfahren nach Anspruch 11, wobei die Reaktion bei einer Temperatur im Bereich von 40°C bis 140°C durchgeführt wird.

16. Verfahren nach Anspruch 11, wobei eine razemische Verbindung von Formel (I) unter Verwendung von 1-(*R*)-oder 1-(*S*)-Camphersulfon-10-säure, (R)- oder (*S*)-Weinsäure, insbesondere 1-(*R*)- oder 1-(*S*)-Camphersulfon-10-säure, in Gegenwart eines Lösungsmittels wie Wasser, Aceton, Ethylacetat oder einer Mischung davon aufgelöst wird.

17. Verfahren nach Anspruch 16, wobei eine Mischung mit verschiedenen Verhältnissen der beiden Enantiomere (-)-I und (+)-I chiral zu dem (+)-(I)-Stereoisomer angereichert wird, oder das (-)-(I)-Enantiomer wird chiral aus einer variablen Mischung von (-)-I- und (+)-1-Stereoisomeren entfernt.

18. Verfahren zum Herstellen einer Verbindung der Formel (IV) oder ihres Salzes, 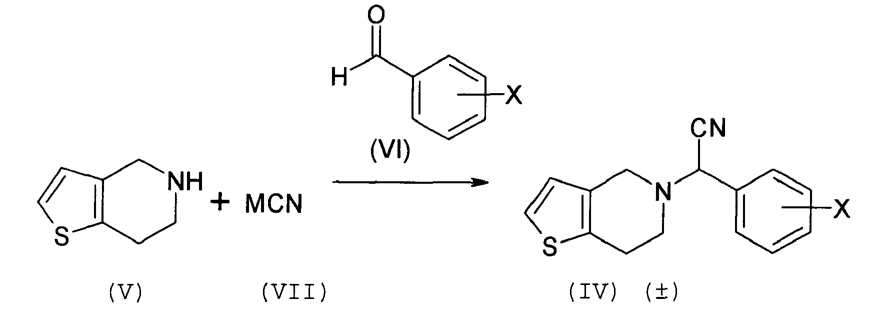 wobei X ein Halogenatom wie Chloro, Bromo oder Iodo, insbesondere 2-Chloro, darstellt, welches umfasst:
i) Umsetzen einer Verbindung von Formel (V) oder ihres Säureadditionssalzes mit einem Cyanid der allgemeinen Formel (VII), wobei die Bedeutung von M Alkalimetall, TMS, Cu oder Wasserstoff ist, gefolgt von der Reaktion mit einem Halogenbenzaldehyd der Formel (VI), wobei X ein Halogenatom ist, und Auflösen des Produktes in seine optischen Isomere und, falls gewünscht, seine Freisetzung aus seinem Salz oder seine Transformation zu seinem Salz, oder
(ii) Umsetzen eines Halogenbenzaldehyds der allgemeinen Formel (VI), wobei die Bedeutung von X Halogenatom ist, mit einem Cyanid der allgemeinen Formel (VII), wobei M wie oben definiert ist, gefolgt von einer *in-situ-*Addition der Verbindung der Formel (V) oder deren Säureadditionssalzes, oder
(iii) Umsetzen eines Halogenbenzaldehyds der allgemeinen Formel (VI), wobei die Bedeutung von X Halogenatom ist, mit einem Wasserstoffsulfit der allgemeinen Formel M'HSO₃ (VIII), wobei M' Natrium, Kalium oder Lithium ist, und schließlich mit einem Cyanid der allgemeinen Formel (VII), wobei M wie oben definiert ist, und anschließendes Umsetzen *in situ* mit der Verbindung der allgemeinen Formel (V) oder deren Säureadditionssalz; und
(iv) schließlich Auflösen der resultierenden Verbindung von Formel (IV) oder deren Salzes zu ihrer optisch reinen (+)- und (-)-Form der Verbindung von Formel (IV) oder ihres Salzes.

19. Verfahren nach Anspruch 18, wobei die Reaktion bei einer Temperatur zwischen 0°C und 100°C durchgeführt wird, außer dass die Reaktionstemperatur weiter bis -30°C sinken kann, wenn es sich bei MCN um HCN-Gas handelt.

20. Verfahren nach Anspruch 18 oder 19, wobei die Reaktion in einem wässrigen oder einem nichtwässrigen Medium durchgeführt wird, welches eine Säure wie beispielsweise Essigsäure oder Propionsäure, Methanol und HC1/MeOH enthält.

21. Verfahren nach einem der Ansprüche 18 bis 20, wobei eine Katalysatorsäure wie beispielsweise trockene Salzsäure, Schwefelsäure, p-Toluolsulfonsäure oder Essigsäure verwendet wird.

22. Verfahren nach einem der Ansprüche 18 bis 21, wobei die Verbindung von Formel (IV), (±)-(2-Chlorphenyl)-(6,7-dihydro-4H-thieno[3,2-c]pyrid-5-yl)acetnitril, in Gegenwart von Aceton, Ethylacetat oder einer Mischung davon und Wasser in ihre (-)- und (+)-Formen aufgelöst wird, wobei 1-(*R*)- oder 1-(*S*)-Camphersulfon-10-säure oder Weinsäure, insbesondere 1-(*R*)- oder 1-(*S*)-Camphersulfon-10-säure verwendet wird.

23. Verfahren nach einem der Ansprüche 18 bis 21, wobei eine Verbindung von Formel (IV), (±)-(2-Chlorphenyl)-(6,7-dihydro-4*H*-thieno[3,2-c]pyrid-5-yl)acetnitril, in (±)- oder (-)- oder (+)-Form in ihr Salz umgewandelt wird, wobei das beanspruchte Salz D-Camphersulfonat, L-Camphersulfonat, D-Tartrat, L-Tartrat oder Hydrogensulfat ist.

24. Verfahren nach einem der Ansprüche 18 bis 23, wobei die Razemisierung des (-)-Isomers der Verbindung von Formel (II) in einer Mischung, welche das (+)-Stereoisomer enthalten kann, um die (±)-Verbindung zu erhalten, unter Verwendung einer Base wie LDA, KOH, NaOH, K-*t*-BuOH, NaOMe, NaH oder KH in einem geeigneten Lösungsmittel durchgeführt wird.

25. Verfahren nach Anspruch 1, wobei die Razemisierung des (-)-Isomers der Verbindung von Formel (I) in einer Mischung, welche das (+)-Stereoisomer enthalten kann, um die (±)-Verbindung zu erhalten, unter Verwendung einer Base wie LDA, NaOMe, NaH, KH in einem geeigneten Lösungsmittel durchgeführt wird.

26. Verfahren nach Anspruch 17, wobei die Auflösung in Gegenwart von Aceton als Lösungsmittel im Bereich von 5 bis 10 Volumen, welches bis zu 5% Wasser enthalten kann, durchgeführt wird.

27. Verfahren nach Anspruch 17, wobei das geeignete chirale Mittel in einem Molverhältnis von 1:1 verwendet wird.

28. Verfahren nach Anspruch 27, wobei die Temperatur von 0°C bis zur Rückflusstemperatur des Lösungsmittels beträgt.

## Revendications

1. Procédé de préparation d'un composé de formule (1), 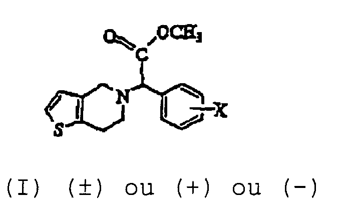 dans laquelle X représente un atome d'hydrogène ou un groupe fluoro, chloro, bromo ou iodo, de préférence 2-chloro, qui comprend les étapes consistant à :
i) faire réagir un composé de formule (V) ou son sel, 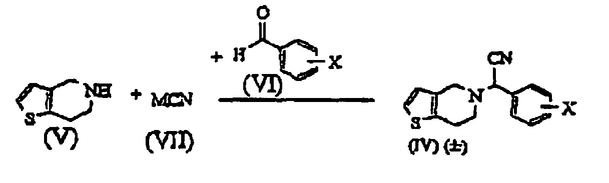 avec un cyanure de formule générale (VII), dans laquelle M représente un métal alcalin, un groupe triméthylsilyle, Cu, ou un atome d'hydrogène, avec l'addition d'un composé de formule générale (VI), dans laquelle X est tel que défini précédemment, pour obtenir un composé racémique (±) de formule générale (IV), dans laquelle X est tel que défini précédemment,
ii) faire réagir un composé de formule générale (IV), sous forme (±) ou sous n'importe laquelle de ses formes optiquement actives (+) ou (-), 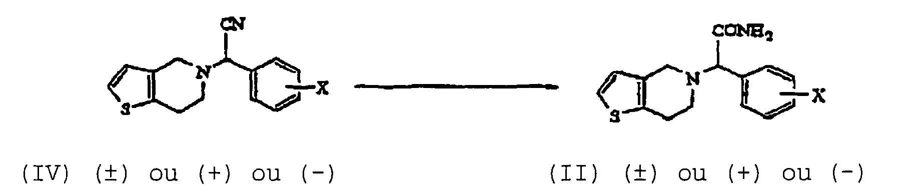 avec un réactif acide ou basique, pour obtenir un composé de formule (II) ou son sel, avec rétention de la configuration,
(iii) faire réagir un composé de formule générale (II), sous forme (±) ou sous ses formes optiquement actives (+) ou (-), 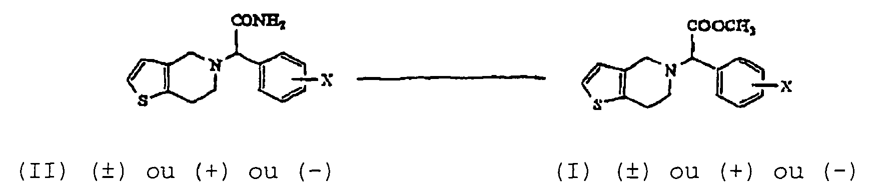 avec un réactif acide en présence de méthanol, pour obtenir un composé de formule (I) ou son sel, avec rétention de la configuration,
(iv) et finalement résoudre le composé de formule (I) ou son sel, s'il est sous forme (±), en ses isomères optiques.

2. Procédé selon la revendication 1, comprenant aussi les étapes consistant à résoudre un composé racémique (±) de formule (IV) en ses isomères optiques et/ou transformer le composé racémique ou son isomère optique en son sel, étape suivie éventuellement de la libération du sel d'avec le composé.

3. Procédé selon la revendication 1, comprenant aussi les étapes consistant à résoudre un composé racémique (±) de formule (II) en ses isomères optiques et/ou transformer le composé racémique ou son isomère optique en son sel, étape suivie éventuellement de la libération du sel d'avec le composé.

4. Procédé de préparation d'un composé de formule (III) ou de son sel, dans lequel X représente un atome d'hydrogène ou un groupe chloro, bromo ou iodo, de préférence 2-chloro, 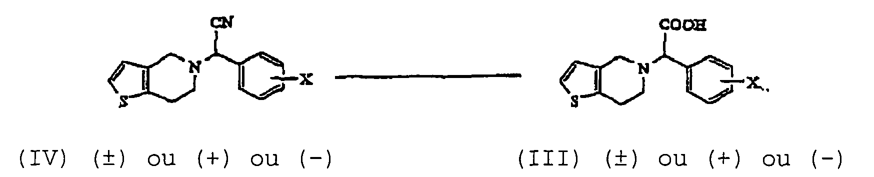 qui comprend les étapes consistant à
(i) préparer un composé de formule générale (IV) ou son sel, sous l'une quelconque de ses formes optiques, par un procédé selon l'étape i) de la revendication 1 ou 2, en présence d'un catalyseur acide/basique dans un solvant approprié, pour conduire à l'acide correspondant de formule (III) ou son sel, avec une configuration identique à celle du composé de départ, et
(ii) le cas échéant, le composé racémique de formule (III) ou son sel, s'il est sous forme racémique (±), est finalement résolu en ses formes optiquement pures (+) et (-) correspondantes.

5. Procédé selon la revendication 1 ou 2, dans lequel un composé de formule (II), un (2-halogénophényl)-(6,7-dihydro-4H-thiéno(3,2-c)pyrid-5-yl)acétamide ou ses isomères optiques actifs ou ses sels, dans lequel X représente un atome d'halogène tel qu'un substituant chloro, bromo, iodo, mieux encore 2-chloro, est préparé par un procédé comprenant les étapes consistant à 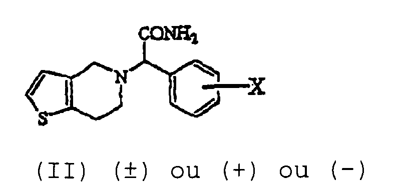
(i) traiter le composé de formule (IV) ou son sel ou l'une quelconque de ses formes optiques, avec un réactif basique lorsque le composé de départ est sous forme racémique, ou bien avec un catalyseur acide lorsque le composé de départ est sous une forme optiquement active, en présence d'un solvant convenable ; et
(ii) finalement former un sel du composé racémique de formule (II), qui est résolu en ses formes optiquement pures (+) ou (-) correspondantes.

6. Procédé selon la revendication 5, qui comprend la préparation d'un composé de formule (II) **caractérisé en ce que** le réactif basique utilisé est l'hydroxyde de lithium, l'hydroxyde de potassium, l'hydroxyde de sodium et le t-butylate de potassium.

7. Procédé selon la revendication 5, qui comprend la préparation d'un composé de formule (II), dans lequel ledit catalyseur acide utilisé est choisi dans le groupe constitué par l'acide acétique, l'acide p-toluènesulfonique, l'acide trifluoroacétique, l'acide chloroacétique, l'acide perchlorique, l'acide formique ou les acides minéraux tels que les halogénures d'hydrogène alcooliques anhydres, ou une solution aqueuse d'acide chlorhydrique, d'acide sulfurique, de HBr ou un mélange de ceux-ci.

8. Procédé selon la revendication 5, 6 ou 7, qui comprend la préparation d'un composé de formule (II) dans lequel le solvant utilisé est de l'eau, un alcool en C₁-C₄, de l'acétone, de l'acide acétique, du diméthylformamide, du THF, du DMSO, du dioxane, du DME ou un mélange de ceux-ci, de préférence un mélange d'eau, de méthanol ou de t-BuOH.

9. Procédé selon l'une quelconque des revendications 5 à 8, dans lequel un composé de formule (II), le (±)-(2-chlorophényl)-(6,7-dihydro-4H-thiéno-[3,2-c]pyrid-5-yl)acétamide, est résolu en ses formes (-) et (+) au moyen d'acide 1-(R) ou 1-(S)-camphosulfonique-10 ou d'acide tartrique, plus particulièrement d'acide 1-(R) ou 1-(S)-camphosulfonique-10, en présence d'un solvant tel que de l'eau, de l'acétone, de l'acétate d'éthyle ou un mélange de ceux-ci, le sel utilisé étant éventuellement sous forme de rapport molaire de 0,5 à 1,1 équivalents.

10. Procédé selon l'une quelconque des revendications 5 à 8, dans lequel le composé de formule (II), le (±)-(2-chlorophényl)-(6,7-dihydro-4H-thiéno-[3,2-c]pyrid-5-yl)acétamide, est transformé en sel correspondant choisi parmi un D-camphosulfonate, un L-camphosulfonate, un D-tartrate, un L-tartrate ou un hydrogénosulfate.

11. Procédé selon l'une quelconque des revendications 1 à 3 ou 5 à 10, dans lequel ledit composé de formule (I) est un (2-halogénophényl)-(6,7-dihydro-4H-thiéno[3,2-c]pyrid-5-yl)acétate de méthyle, ou ses isomères optiques actifs ou ses sels, où X représente un atome d'halogène tel qu'un substituant chloro, bromo, iodo, mieux encore 2-chloro, 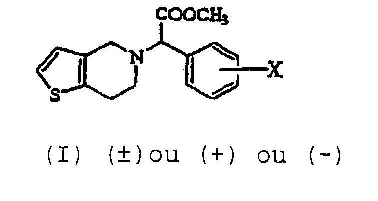 ledit procédé comprenant les étapes consistant à
(i) traiter le composé de formule (II) ou son sel sous forme racémique ou sous l'une quelconque de ses formes optiques, avec un réactif acide en présence d'un solvant convenable ; et
(ii) former un sel du composé racémique de formule (I), qui est résolu en ses formes optiquement pures (+) ou (-) correspondantes.

12. Procédé selon la revendication 11, dans lequel ledit réactif acide utilisé est choisi parmi l'acide sulfurique concentré à raison de 1 équivalent à 50 équivalents, de préférence à une température de 0°C à 5°C ou à la température de reflux du solvant utilisé.

13. Procédé selon la revendication 11, dans lequel le solvant utilisé est du méthanol à raison de 3 à 30 volumes, mélangé avec un cosolvant tel que le toluène, le DMSO ou le xylène, ou un mélange de ceux-ci.

14. Procédé selon la revendication 11, dans lequel la durée du traitement avec un réactif acide s'échelonne de 4 heures à 4 jours.

15. Procédé selon la revendication 11, dans lequel la réaction est mise en oeuvre à une température sur la gamme de 40°C à 140°C.

16. Procédé selon la revendication 11, dans lequel un composé racémique de formule (I) est résolu au moyen d'acide 1-(R) ou 1-(S)-camphosulfonique-10, d'acide (R) ou (S)-tartrique, plus particulièrement d'acide 1-(R) ou 1-(S)-camphosulfonique-10, en présence d'un solvant tel que de l'eau, de l'acétone, de l'acétate d'éthyle ou un mélange de ceux-ci.

17. Procédé selon la revendication 16, dans lequel un mélange contenant différents rapports des deux énantiomères, (-)-I et (+)-I, est enrichi sur le plan chiral en stéréoisomère (+)-(I), ou l'énantiomère (-)-I est séparé sur le plan chiral d'un mélange variable de stéréoisomères (-)-I et (+)-I.

18. Procédé de préparation d'un composé de formule (IV) ou de son sel, 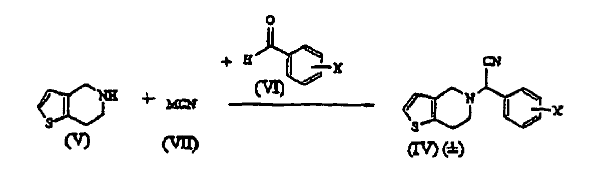 dans lequel X représente un atome d'halogène tel que chloro, bromo ou iodo, plus particulièrement 2-chloro, qui comprend les étapes consistant à
i) faire réagir un composé de formule (V) ou son sel d'addition d'acide avec un cyanure de formule générale (VII), dans laquelle M désigne un métal alcalin, TMS, Cu et un atome d'hydrogène, puis effectuer une réaction avec un halogénobenzaldéhyde de formule (VI), dans laquelle X est un atome d'halogène, et résoudre le produit en ses isomères optiques et, le cas échéant, le libérer de son sel ou le transformer en son sel ; ou
ii) faire réagir un halogénobenzaldéhyde de formule générale (VI), dans laquelle la signification de X est un atome d'halogène, avec un cyanure de formule générale (VII), dans laquelle M est tel que défini précédemment, réaction suivie d'une addition in situ du composé de formule (V) ou de son sel d'addition d'acide ; ou
iii) faire réagir un halogénobenzaldéhyde de formule générale (VI), dans laquelle la signification de X est un atome d'halogène, avec un hydrogénosulfite de formule générale M'HSO₃ (VIII), dans laquelle M' est un sodium, un potassium ou un lithium, et finalement avec un cyanure de formule générale (VII), dans laquelle M est tel que défini précédemment, et ensuite effectuer une réaction in situ avec le composé de formule générale (V) ou son sel d'addition d'acide ; et
iv) finalement résoudre le composé de formule (IV) ainsi obtenu ou son sel en formes (+) et (-) optiquement pures du composé de formule (IV) ou de son sel.

19. Procédé selon la revendication 18, dans lequel la réaction est mise en oeuvre à une température comprise entre 0°C et 100°C, sauf que, lorsque MCN est du gaz HCN, la température réactionnelle peut descendre encore jusqu'à -30°C.

20. Procédé selon la revendication 18 ou 19, dans lequel la réaction est effectuée dans un milieu aqueux ou non aqueux contenant un acide tel que l'acide acétique ou l'acide propionique, du méthanol et HCl/MeOH.

21. Procédé selon l'une quelconque des revendications 18 à 20, dans lequel un catalyseur acide, tel que de l'acide chlorhydrique anhydre, de l'acide sulfurique, de l'acide p-toluènesulfonique ou de l'acide acétique, est utilisé.

22. Procédé selon l'une quelconque des revendications 18 à 21, dans lequel ledit composé de formule (IV), le (±)-(2-chlorophényl)-(6,7-dihydro-4H-thiéno[3,2-c]pyrid-5-yl)acétonitrile, est résolu en formes (-) et (+) au moyen d'acide 1(R)- ou 1(S)-camphosulfonique-10 ou d'acide tartrique, plus particulièrement d'acide 1(R) ou 1(S)-camphosulfonique-10, en présence d'acétone, d'acétate d'éthyle ou d'un mélange de ceux-ci et d'eau.

23. Procédé selon l'une quelconque des revendications 18 à 21, dans lequel un composé de formule (IV), le (±)-(2-chlorophényl)-(6,7-dihydro-4H-thiéno-[3,2-c]pyrid-5-yl)acétonitrile, sous forme (±) ou (-) ou (+), est transformé en son sel, dans lequel le sel revendiqué est un D-camphosulfonate, un L-camphosulfonate, un D-tartrate, un L-tartrate ou un hydrogénosulfate.

24. Procédé selon l'une quelconque des revendications 18 à 23, dans lequel la racémisation de l'isomère (-) du composé de formule (II) s'effectue dans un mélange qui peut contenir un stéréoisomère (+) pour donner le composé (±) en utilisant une base telle que LDA, KOH, NaOH, K-t-BuOH, NaOMe, NaH ou KH dans un solvant approprié.

25. Procédé selon la revendication 1, dans lequel la racémisation de l'isomère (-) du composé de formule (I) s'effectue dans un mélange qui peut contenir un stéréoisomère (+) pour donner le composé (±) en utilisant une base telle que LDA, NaOMe, NaH, KH dans un solvant approprié.

26. Procédé selon la revendication 17, dans lequel la résolution est mise en oeuvre en présence d'acétone comme solvant à raison de 5 à 10 volumes, qui peut contenir de l'eau jusqu'à 5%.

27. Procédé selon la revendication 17, dans lequel ledit agent chiral approprié utilisé est dans un rapport molaire de 1:1.

28. Procédé selon la revendication 27, dans lequel la température va de 0°C à la température de reflux du solvant.
